# EUROPEAN PATENT APPLICATION

(11) **EP 1 762 553 A1**
(43) Date of publication of application: **14.03.2007**
(21) Application number: 05753256.6
(22) Date of filing: 23.06.2005
(51) Int. Cl.: C07C 13/66, C07C 13/72, C07D 209/86, C09K 11/86, H05B 33/14

(54) **POLYCYCLIC AROMATIC COMPOUND, MATERIAL FOR FORMING LUMINESCENT COATING FILM AND ORGANIC ELECTROLUMINESCENT DEVICE USING SAME**

(30) Priority: 28.06.2004 JP 2004189367
(71) Applicant: IDEMITSU KOSAN CO., LTD., Tokyo 100-8321 (JP)
(72) Inventor: INOUE, Tetsuya, 2990293 (JP); KAWAMURA, Hisayuki, 2990293 (JP); ITO, Mitsunori, 2990293 (JP); HOSOKAWA, Chishio, 2990293 (JP)
(74) Representative: Gille Hrabal Struck Neidlein Prop Roos
(86) International application number: PCT/JP2005/011537
(87) International publication number: WO 2006/001333

(57) **Abstract**

Disclosed are a novel polycyclic aromatic compound having a specific structure and a material for forming a luminescent coating film which is composed of an organic solvent solution containing such a polycyclic aromatic compound. Also disclosed is an organic electroluminescent (EL) device wherein an organic thin film layer composed of one or more layers including at least a light-emitting layer is interposed between a cathode and an anode. Such an organic EL device has high luminous efficiency and long life, since the polycyclic aromatic compound is contained, by itself or as a component of a mixture, in at least one layer of the organic thin film layer of the organic EL device.

## Description

### TECHNICAL FIELD

The present invention relates to a polycyclic aromatic compound and an organic electroluminescence (EL) device using the compound, in particular, an organic EL device having high luminous efficiency and a long lifetime, and a novel polycyclic aromatic compound and a novel material for forming a luminescent coating film each realizing the organic EL device.

### BACKGROUND ART

An organic electroluminescent device is a spontaneous light emitting device which utilizes a principle that a fluorescent substance emits light by energy of recombination of holes injected from an anode and electrons injected from a cathode when an electric field is applied. Since an organic EL device of the laminate type driven under low electric voltage was reported by C. W. Tang et al. Eastman Kodak Co. (C. W. Tang and S. A. Vanslyke, Applied Physics Letters, Volume 51, P. 913, 1987) , many studies have been conducted on organic EL devices using organic materials as the constituting components. Tang et al. used tris(8-quinolinolato)aluminum for a light emitting layer and a triphenyldiamine derivative for a hole transporting layer. Advantages of the laminate structure are that the efficiency of hole injection into the light emitting layer can be increased, the efficiency of forming exciton which are formed by blocking and recombining electrons injected from the cathode can be increased, exciton formed within the light emitting layer can be enclosed, and the like. For the structure of the organic EL device as in the example, a two-layered structure having a hole transporting (or injecting) layer and an electron transporting and light emitting layer, a three-layered structure having a hole transporting (or injecting) layer, a light emitting layer, and an electron transporting (or injecting) layer, and the like are well known. To increase the efficiency of recombination of injected holes and electrons in the devices having such the laminate type structures, the structure of the device and the process for forming the device have been modified.

Known examples of a light emitting material include: a chelate complex such as a tris (8-quinolinolato) aluminum complex; a coumarin derivative; a tetraphenylbutadiene derivative; a bisstyrylarylene derivative; and an oxadiazole derivative. It has been reported that light in a visible region ranging from a blue color to a red color can be emitted from each of those light emitting materials, so the realization of a color display device has been expected from each of them (for example, Patent Document 1, Patent Document 2, and Patent Document 3).
In addition, Patent Document 4 discloses a device using a phenylanthracene derivative as a light emitting material. Further, Patent Document 5 discloses a material obtained by providing naphthyl groups for 9- and 10-positions of anthracene . Each of such anthracene derivatives has been used as a blue light emitting material. However, an improvement in lifetime of a device using any one of the derivatives has been requested.
Further, Patent Document 6 discloses a material obtained by providing fluoranthene groups for 9- and 10-positions of anthracene. Such anthracene derivative has been used as a blue light emitting material. However, an improvement in lifetime of a device using the derivative has been requested.
In recent years, for example, Patent Document 7, Patent Document 8, Patent Document 9, and Patent Document 10 have disclosed light emitting materials each having a fluorene skeleton. However, the performance of a device using any one of those materials is not sufficient for practical use in terms of both efficiency and lifetime, so an improvement in the performance has been requested.
There has been also proposed that a phosphorescent material as well as a fluorescent material is used in a light emitting layer of an organic EL device (see, for example, Non-patent Document 1 and Non-patent Document 2).
High luminous efficiency is achieved by utilizing a singlet state and a triplet state in excited states of a phosphorescent material in a light emitting layer of an organic EL device in this way. When an electron and a hole recombine in the organic EL device, singlet excitons and triplet excitons are considered to be produced at a ratio of 1:3 owing to a difference in spin multiplicity. Accordingly, the use of a phosphorescent light emitting material is considered to achieve luminous efficiency three to four times as high as that of a device using only a fluorescent material.
A constitution in which layers are sequentially laminated, for example, an organic light emitting layer, an electron transporting layer (hole blocking layer), an electron injecting layer, and a cathode are laminated in the stated order is used in such organic EL device in order that a triplet excited state or a triplet exciton should not quench. A host compound and a phosphorescent compound are used in the organic light emitting layer (see, for example, Patent Document 11 and Patent Document 12). 4,4-N,Ndicarbazolebiphenyl has been used as the host compound. However, the compound has a glass transition temperature of 110°C or lower, and its symmetry is so good that the compound is apt to crystallize. Inaddition, there arises a problem in that the compound causes a short circuit or an image defect when a heat resistance test is performed for the device. The following has been also found: when the compound is deposited from the vapor, crystal growth occurs at, for example, a site where foreign matter or a projection of an electrode is present, so defects are generated even in an initial state before the heat resistance test. Further, a carbazole derivative having three-fold symmetry has been also used as a host. However, the derivative also has high crystallinity, so the following situation inevitably occurs: when the derivative is deposited from the vapor, crystal growth occurs at, for example, a site where foreign matter or a projection of an electrode is present, so defects are generated even in an initial state before a heat resistance test.

Patent Document 1: JP-A-08-239655
Patent Document 2: JP-A-07-183561
Patent Document 3: JP-A-03-200289
Patent Document 4: JP-A-08-012600
Patent Document 5: JP-A-11-003782
Patent Document 6: JP-A-2001-257074
Patent Document 7: JP-A-2002-326965
Patent Document 8: JP-A-2004-002298
Patent Document 9: JP-A-2004-083481
Patent Document 10: JP-A-2004-083483
Patent Document 11: US 6,097,147
Patent Document 12: WO 01/41512 A
Non-patent Document 1: D. F. OBrien and M. A. Baldoetal, "Improved energy transfer in electrophosphorescent devices", Applied Physics letters, Vol. 74, No. 3, pp 442-444, January 18, 1999
Non-patent Document 2: M. A. Baldoetal, "Very high-efficiency green organic light emitting devices based on electrophasphorescence", Applied Physics letters, Vol. 75, No. 1, pp 4-6, July 5, 1999

### DISCLOSURE OF THE INVENTION

The present invention has beenmade with a view to solving the above-mentioned problems, and an object of the present invention is to provide an organic EL device having high luminous efficiency and a long lifetime, and a polycyclic aromatic compound and a material for forming a luminescent coating film each realizing the organic EL device.

The inventors of the present invention have made extensive studies with a view to achieving the above-mentioned obj ect. As a result, the inventors of the present invention have found that the use of a polycyclic aromatic compound represented by the following general formula (1) as a material for an organic EL device achieves the above-mentioned object, thereby completing the present invention.

That is, the present invention provides a polycyclic aromatic compound represented by the following general formula (1) :

X-Y-X' (1)

where:
X and X' each independently represent a substituted or unsubstituted aromatic group having 6 to 50 carbon atoms, or a substituted or unsubstituted aromatic heterocyclic group having 5 to 50 ring atoms, and may be identical to or different from each other; and
Y represents a divalent group containing at least one linking group represented by the following general formula (2) or (3):

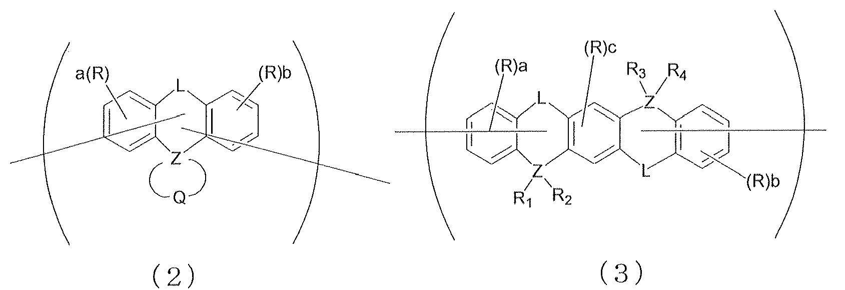

where:
R's and R₁ to R₄ each independently represent a substituted or unsubstituted aromatic group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 7 to 50 carbon atoms, a substituted or unsubstituted aryloxy group having 5 to 50 ring atoms, a substituted or unsubstituted arylthio group having 5 to 50 ring atoms, a carboxyl group, a halogen atom, a cyano group, a nitro group, or a hydroxyl group, and R₁ and R₂, or R₃ and R₄ may be bonded to each other to form a cyclic structure;
L's each represent a single bond, - (CR' R")_{c}-, -(SiR'R")_{c}-, -O-, -CO-, or -NR'- where R' and R" each independently represent a hydrogen atom, a substituted or unsubstituted aromatic group having 6 to 50 ring carbon atoms, a substituted or unsubstituted aromatic heterocyclic group having 5 to 50 ring atoms, or a substituted or unsubstituted alkyl group having 1 to 50 carbon atoms, c represents an integer of 1 to 10, and R' and R" may be identical to or different from each other;
Z's each represent a carbon atom, a silicon atom, or a germanium atom;
Q represents a cyclic structure forming group; and
a and b each represent an integer of 0 to 4, and c represents an integer of 0 to 2.

The present invention further provides a material for forming a luminescent coating film, the material including an organic solvent solution containing the polycyclic aromatic compound, and an organic electroluminescence device which includes an organic thin film layer composed of one or multiple layers including at least a light emitting layer, the organic thin film layer being interposed between a cathode and an anode, in which at least one layer of the organic thin film layer contains the polycyclic aromatic compound alone or as a component of a mixture.

The organic electroluminescence device using the polycyclic aromatic compound and the material for forming a luminescent coating film of the present invention has high luminous efficiency and a long lifetime.

### BEST MODE FOR CARRYING OUT THE INVENTION

The polycyclic aromatic compound of the present invention is a compound represented by the following general formula (1).

X-Y-X' (1)

where:
X and X' each independently represent a substituted or unsubstituted aromatic group having 6 to 50 carbon atoms, or a substituted or unsubstituted aromatic heterocyclic group having 5 to 50 ring atoms, and may be identical to or different from each other.

Examples of the substituted or unsubstituted aromatic group having 6 to 50 carbon atoms represented by each of X and X' include a phenyl group, a 2-biphenylyl group, a 3-biphenylyl group, a 4-biphenylyl group, a terphenylyl group, a 3, 5-diphenylphenyl group, a 3,4-diphenylphenyl group, a pentaphenylphenyl group, a 4-(2,2-diphenylvinyl)phenyl group, a 4--(1,2,2-triphenylvinyl)phenyl group, a fluorenyl group, a 1-naphthyl group, a 2-naphthyl group, a 9-anthryl group, a 2-anthryl group, a 9-phenanthryl group, a 1-pyrenyl group, a chrysenyl group, a naphthacenyl group, and a coronyl group.

Examples of the substituted or unsubstituted aromatic heterocyclic group having 5 to 50 ring atoms represented by each of X and X' include a carbazolyl group, a dibenzofuranyl group, a dibenzothiophenyl group, a fluorenonyl group, an oxazolyl group, an oxadiazolyl group, a thiadiazole group, and a benzoimidazole group.

In addition, in the polycyclic aromatic compound of the present invention, at least one of X and X' preferably represents a pyrenyl group or a chrysenyl group.

In the general formula (1), Y represents a divalent group containing at least one linking group represented by the following general formula (2) or (3).

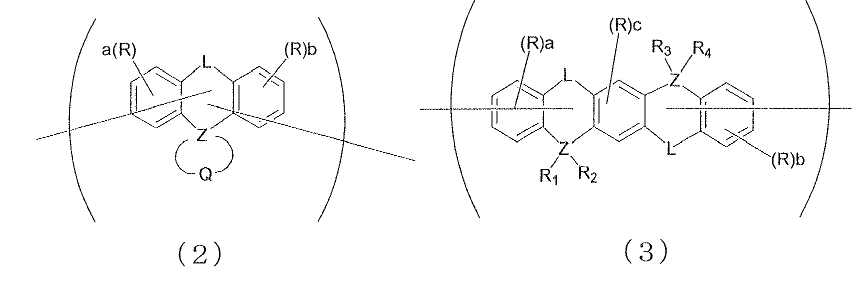

In the general formulae (2) and (3), R's and R₁ to R₄ each independently represent a substituted or un substituted aromatic group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 7 to 50 carbon atoms, a substituted or unsubstituted aryloxy group having 5 to 50 ring atoms, a substituted or unsubstituted arylthio group having 5 to 50 ring atoms, a carboxyl group, a halogen atom, a cyano group, a nitro group, or a hydroxyl group.

Examples of the substituted or unsubstituted aromatic group having 6 to 50 ring carbon atoms represented by any one of R's and R₁ to R₄ include a phenyl group, a 2-biphenylyl group, a 3-biphenylyl group, a 4-biphenylyl group, a terphenylyl group, a 3,5-diphenylphenyl group, a 3,4-diphenylphenyl group, pentaphenylphenyl group, a 4-(2,2-diphenylvinyl)phenyl group, a 4-(1,2,2-triphenylvinyl)phenyl group, a fluorenyl group, a 1-naphthyl group, a 2-naphthyl group, a 9-anthryl group, a 2-anthryl group, a 9-phenanthryl group, a 1-pyrenyl group, a crycenyl group, a naphthacenyl group, and a coronyl group.

Examples of the substituted or unsubstituted aromatic heterocyclic group having 5 to 50 ring atoms represented by any one of R's and R₁ to R₄ include a 1-pyrrolyl group, a 2-pyrrolyl group, a 3-pyrrolyl group, a pyrazinyl group, a 2-pyrizinyl group, a 3-pyridinyl group, a 4-pyridinyl group, a 1-indolyl group, a 2-indolyl group, a 3-indolyl group, a 4-indolyl group, a 5-indolyl group, a 6-indolyl group, a 7-indolyl group, a 1-isoindolyl group, a 2-isoindolyl group, a 3-isoindolyl group, a 4-isoindolyl group, a 5-isoindolyl group, a 6-isoindolyl group, a 7-isoindolyl group, a 2-furyl group, a 3-furyl group, a 2-benzofuranyl group, a 3-benzofuranyl group, a 4-benzofuranylgroup, a 5-benzofuranylgroup, a 6-benzofuranyl group, a 7-benzofuranyl group, a 1-isobenzofuranyl group, a 3-isobenzofuranyl group, a 4-isobenzofuranyl group, a 5-isobenzofuranyl group, a 6-isobenzofuranyl group, a 7-isobenzofuranyl group, a quinolyl group, a 3-quinolyl group, a 4-quinolyl group, a 5-quinolyl group, a 6-quinolyl group, a 7-quinolyl group, an 8-quinolyl group, a 1-isoquinolyl group, a 3-isoquinolyl group, a 4-isoquinolyl group, a 5-isoquinolyl group, a 6-isoquinolyl group, a 7-isaquinolyl group, an 8-isoquinolyl group, a 2-quinoxalinyl group, a 5-quinoxalinyl group, a 6-quinoxalinyl group, a 1-carbazolyl group, a 2-carbazolyl group, a 3-carbazolyl group, a 4-carbazolyl group, a 9-carbazolyl group, a 1-phenanthridinyl group, a 2-phenanthridinyl group, a 3-phenanthridinyl group, a 4-phenanthridinyl group, a 6-phenanthridinyl group, a 7-phenanthridinyl group, an 8-phenanthridinyl group, a 9-phenanthridinyl group, a 10-phenanthridinyl group, a 1-acridinyl group, a 2-acridinyl group, a 3-acridinyl group, a 4-acridinyl group, a 9-acridinyl group, a 1,7-phenanthroline-2-yl group, a 1,7-phenanthroline-3-yl group, a 1,7-phenanthroline-4-yl group, a 1,7-phenanthroline-5-yl group, a 1,7-phenanthroline-6-yl group, a 1,7-phenanthroline-8-yl group, a 1,7-phenanthroline-9-yl group, a 1, 7-phenanthroline-10-yl group, a 1,8-phenanthroline-2-yl group, a 1,8-phenanthroline-3-yl group, a 1,8-phenanthroline-4-yl group, a 1,8-phenanthroline-5-yl group, a 1,8-phenanthroline-6-yl group, a 1,8-phenanthroline-7-yl group, a 1, 8-phenanthroline-9-yl group, a 1,8-phenanthroline-10-yl group, a 1,9-phenanthroline-2-yl group, a 1,9-phenanthroline-3-yl group, a 1, 9-phenanthroline-4-yl group, a 1, 9-phenanthroline-5-yl group, a 1, 9-phenanthroline-6-yl group, a 1,9-phenanthroline-7-yl group, a 1, 9-phenanthroline-8-yl group, a 1, 9-phenanthroline-10-yl group, a 1, 10-phenanthroline-2-yl group, a 1, 10-phenanthraline-3-yl group, a 1, 10-phenanthroline-4-yl group, a 1, 10-phenanthroline 5-yl group, a 2, 9-phenanthroline 1-yl group, a 2, 9-phenanthroline-3-yl group, a 2, 9-phenanthroline-4-yl group, a 2, 9-phenanthroline-5-yl group, a 2, 9-phenanthroline-6-yl group, a 2, 9-phenanthroline-7-yl group, a 2,9-phenanthroline-8-yl group, a 2,9-phenanthroline-10-yl group, a 2, 8-phenanthroline-1-yl group, a 2, 8-phenanthroli-ne-3-yl group, a 2, 8-phenanthroline-4-yl group, a 2, 8-phenanthroline-5-yl group, a 2, 8-phenanthroline-6-yl group, a 2, 8-phenanthroline-7-yl group, a 2, 8-phenanthroline-9-yl group, a 2, 8-phenanthroline-10-yl group, a 2,7-phenanthroline-1-yl group, a 2, 7-phenanthroline-3-yl group, a 2, 7-phenanthroline-4-yl group, a 2, 7-phenanthroline-5-yl group, a 2,7-phenanthroline-6-yl group, a 2, 7-phenanthroline-8-yl group, a 2,7-phenanthroline-9-ylgroup, a 2, 7-phenanthroline-10-yl group, a 1-phenazinyl group, a 2-phenazinyl group, a 1-phenothiazinyl group, a 2-phenothiazinyl group, a 3-phenothiazinyl group, a 4-phenothiazinyl group, a 10-phenothiazinyl group, a 1-phenoxadinyl group, a 2-phenoxadinyl group, a 3-phenoxadinyl group, a 4-phenoxadinyl group, a 10-phenoxadinyl group, a 2-oxazolyl group, a 4-oxazolyl group, a 5-oxazolyl group, a 2-oxadiazolyl group, a 5-oxadiazolyl group, a 3-furazanyl group, a 2-thienyl group, a 3-thienyl group, a 2-methylpyrrol-1-yl group, a 2-methylpyrrol-3-yl group, a 2-methylpyrrol-4-yl group, a 2-methylpyrrol-5-yl group, a 3-methylpyrrol-1-yl group, a 3-methylpyrrol-2-yl group, a 3-methylpyrrol-4-yl group, a 3-methylpyrrol-5-yl group, a 2-t-butylpyrrol-4-yl group, a 3- (2-phenylpropyl) pyrrol-1-yl group, a 2-methyl-1-indolyl group, a 4-methyl-1-indolyl group, a 2-methyl-3-indolyl group, a 4-methyl-3-indolyl group, a 2-t-butyl-1-indolyl group, a 4-t-butyl-1-indolyl group, a 2-t-butyl-3-indolyl group, and a 4-t-butyl-3-indolyl group.

Examples of the substituted or unsubstituted alkyl group having 1 to 50 carbon atoms represented by any one of R's and R₁ to R₄ include a methyl group, an ethyl group, a propyl group, an isopropyl group, an n-butyl group, an s-butyl group, an isobutyl group, a t-butyl group, an n-pentyl group, an n-hexyl group, an n-heptyl group, an n-octyl group, a hydroxymethyl group, a 1-hydroxyethyl group, a 2-hydroxyethyl group, a 2-hydroxyisobutyl group, a 1,2-dihydroxyethyl group, a 1,3-dihydroxyisopropyl group, a 2,3-dihydroxy-t-butyl group, a 1,2,3-trihydroxypropyl group, a chloromethyl group, a 1-chloroethyl group, a 2-chloroethyl group, a 2-chloroisobutyl group, a 1,2-dichloroethyl group, a 1,3-dichloroisopropyl group, a 2,3-dichloro-t-butyl group, a 1,2,3-trichloropropyl group, a bromomethyl group, a 1-bromoethyl group, a 2-bromoethyl group, a 2-bromoisobutyl group, a 1,2-dibromoethyl group, a 1,3-dibromoisopropyl group, a 2,3-dibromo-t-butyl group, a 1,2,3-tribromapropyl group, an iodomethyl group, a 1-iodoethyl group, a 2-iodoethyl group, a 2-iodoisobutyl group, a 1,2-diiodoethyl group, a 1,3-diiodoisopropyl group, a 2,3-diiodo-t-butyl group, a 1,2,3-triiodopropyl group, an aminomethyl group, a 1-aminoethyl group, a 2-aminoethyl group, a 2-aminoisobutyl group, a 1,2-diaminoethyl group, a 1,3-diaminoisopropyl group, a 2,3-diamino-t-butyl group, a 1,2,3-triaminopropyl group, a cyanomethyl group, a 1-cyanoethyl group, a 2-cyanoethyl group, a 2-cyanoisobutyl group, a 1,2-dicyanoethyl group, a 1,3-dicyanoisopropyl group, a 2,3-dicyano-t-butyl group, a 1,2,3-tricyanopropyl group, a nitromethyl group, a 1-nitroethyl group, a 2-nitroethyl group, a 2-nitroisobutyl group, a 1,2-dinitroethyl group, a 1,3-dinitroisopropyl group, a 2,3-dinitro-t-butyl group, a 1,2,3-trinitropropyl group, a cyclopropyl group, a cyclobutyl group, a cyclopentyl group, a cyclohexyl group, a 4-methylcyclohexyl group, a 1-adamantyl group, a 2-adamantyl group, a 1-norbornyl group, and a 2-norbornyl group.

The substituted or unsubstituted alkoxy group having 1 to 50 carbon atoms represented by any one of R's and R₁ to R₄ is a group represented by -OY', and examples of Y' include examples similar to those exemplified for the alkyl group.

Examples of the substituted or unsubstituted aralkyl group having 7 to 50 carbon atoms represented by any one of R's and R₁ to R₄ include a benzyl group, a 1-phenylethyl group, a 2-phenylethyl group, a 1-phenylisopropyl group, a 2-phenylisopropyl group, a phenyl-t-butyl group, an α-naphthylmethyl group, a 1-α-naphthylethyl group, a 2-α-naphthylethyl group, a 1-α-naphthylisopropyl group, a 2-α-naphthylisoprapyl group, a β-naphthylmethyl group, a 1-β-naphthylethyl group, a 2-β-naphthylethyl group, a 1-β-naphthylisopropyl group, a 2-β-naphthylisopropyl group, a 1-pyrrolylmethyl group, a 2-(1-pyrrolyl)ethyl group, a p-methylbenzyl group, an m-methylbenzyl group, an o-methylbenzyl group, a p-chlorobenzyl group, an m-chlorobenzyl group, an o-chlorobenzyl group, a p-bromobenzyl group, an m-bromobenzyl group, an o-bromobenzyl group, a p-iodobenzyl group, an m-iodobenzyl group, an o-iodobenzyl group, a p-hydroxybenzyl group, an m-hydroxybenzyl group, an o-hydroxybenzyl group, a p-aminobenzyl group, anm-aminobenzyl group, an o-aminobenzyl group, a p-nitrobenzyl group, an m-nitrobenzyl group, an o-nitrobenzyl group, a p-cyanobenzyl group, an m-cyanobenzyl group, an o-cyanobenzyl group, a 1-hydroxy-2-phenylisopropyl group, and a 1-chloro-2-phenylisopropyl group.

The substituted or unsubstituted aryloxy group having 5 to 50 ring atoms represented by any one represented by R's and R₁ to R₄ is a group represented by -OY" while the substituted or unsubstituted arylthio group having 5 to 50 ring atoms represented by any one of them is a group represented by -SY". Examples of Y" include examples similar to those exemplified for the aromatic group.

Examples of the halogen atom represented by any one of R's and R₁ to R₄ include fluorine, chlorine, bromine, and iodine.
In addition, R₁ and R₂, and R₃ and R₄ each may be bonded to each other to form a cyclic structure in the general formulae (2) and (3). Examples of the cyclic structure include: cycloalkane having 4 to 12 carbon atoms such as cyclobutane, cyclopentanes, cyclohexane, adamantane, and norbornane; cycloalkenes having 4 to 12 carbon atoms such as cyclobutene, cyclopentene, cyclohexene, cycloheptene, and cyclooctene; cycloalkadienes having 6 to 12 carbon atoms such as cyclohexadiene, cycloheptadiene, and cyclooctadiene; and an aromatic ring having 6 to 50 carbon atoms such as benzene, naphthalene, phenanthrene, anthracene, pyrene, chrysene, and acenaphthylene.

In the general formulae (2) and (3), a and b each represent an integer of 0 to 4, and c represents an integer of 0 to 2.
In the general formulae (2) and (3), L' s each represent a single bond, - (CR'R")_{c}-, - (SiR'R")_{c}-, -O-, -CO-, or -NR'- where R' and R" each independently represent a hydrogen atom, a substituted or unsubstituted aromatic group having 6 to 50 ring carbon atoms, a substituted or unsubstituted aromatic heterocyclic group having 5 to 50 ring atoms, or a substituted or unsubstituted alkyl group having 1 to 50 carbon atoms, c represents an integer of 1 to 10, and R' and R" may be identical to or different from each other.
Examples of the aromatic group represented by R' or R" include examples similar to those exemplified for Y.
In the general formulae (2) and (3), Z's each represent a carbon atom, a silicon atom, or a germanium atom.
In the general formulae (2) and (3) , Q' s each represent a cyclic structure-forming group. Examples of the cyclic structure to be formed by Z-Q include: cycloalkanes having 4 to 12 carbon atoms such as cyclobutane, cyclopentane, cyclohexane, adamantane, and norbornane; cycloalkenes having 4 to 12 carbon atoms such as cyclobutene, cyclopentene, cyclohexene, cycloheptene, and cyclooctene; cycloalkadienes having 6 to 12 carbon atoms such as cyclohexadiene, cycloheptadiene, and cyclooctadiene; and an aromatic ring having 6 to 50 carbon atoms such as benzene, naphthalene, phenanthrene, anthracene, pyrene, chrysene, and acenaphthylene.

The polycyclic aromatic compound of the present invention is preferably such that, in the general formula (1), at least one of X and X' represents a substituted or unsubstituted aromatic group having 16 to 50 carbon atoms, and Y represents a divalent group containing at least one linking group represented by any one of the following general formulae (4) to (6).
Examples of the substituted or unsubstituted aromatic group having 16 to 50 carbon atoms represented by at least one of X and X' include a 1-pyrenyl group, a chrysenyl group, a naphthacenyl group, and a coronyl group. Of those, a 1-pyrenyl group and a chrysenyl group are preferable.

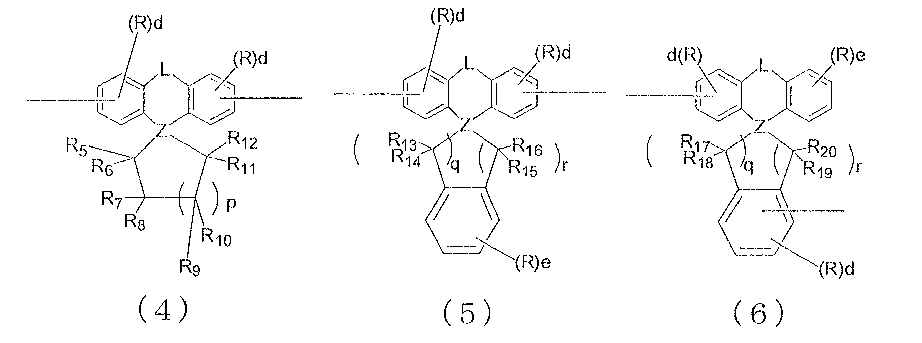

In the general formulae (4) to (6), R's and R₅ to R₂₀ each independently have the same meaning as that of each of R's and R₁ to R₄ described above, and specific examples of R's and R₅ to R₂₀ include examples similar to those of R's and R₁ to R₄ described above.
In addition, two arbitrary adjacent groups among R₅ to R₂₀ may be bonded to each other to form a cyclic structure, and examples of the cyclic structure include examples similar to those described for a combination of R₁ and R₂ and a combination of R₃ and R₄.
In the general formulae (4) to (6), L and Z each independently have the same meaning as that described above.
In the general formulae (4) to (6), d' seach represent an integer of 0 to 3, and e represents an integer of 0 to 4.
In the general formulae (4) to (6), p, q, and r each represent an integer of 1 to 10.

In addition, the polycyclic aromatic compound of the present invention is preferably such that, in the general formula (1), Y represents a divalent group containing at least one linking group represented by the following general formula (7).

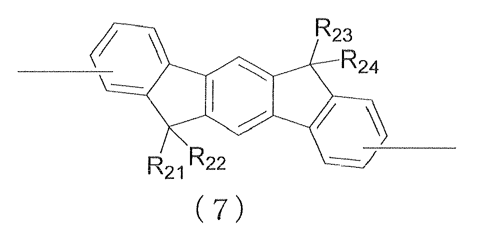

In the general formula (7), R₂₁ to R₂₄ each independently have the same meaning as that of each of R's and R₁ to R₄ described above, and specific examples of R₂₁ to R₂₄ include examples similar to those of R's and R₁ to R₄ described above.
In addition, R₂₁ and R₂₂, or R₂₃ and R₂₄ may be bonded to each other to form a cyclic structure, and examples of the cyclic structure include examples similar to those described for a combination of R₁ and R₂ and a combination of R₃ and R₄.

In addition, the polycyclic aromatic compound of the present invention is preferably such that, in the general formula (1), Y represents a linking group represented by the following general formula (8) or (8').

-(Ar₁)ₖ-L₁-(Ar₂)ₘ-(L₂)ₙ- (8)

- (Ar₁)ₖ-L₁'- (Ar₂)ₘ- (L₂')ₙ- (8')

In the general formulae (8) and (8'), Ar₁ and Ar₂ each independently represent a substituted or unsubstituted, divalent aromatic group having 6 to 12 carbon atoms, and examples of the aromatic group include those obtained by turning the examples exemplified for X and X' into divalent groups.
In the general formula (8), L₁ and L₂ each represent a linking group represented by any one of the general formulae (4) to (6), and L₁ and L₂ may be identical to or different from each other.
In the general formula (8') , L₁' and L₂' each represent a linking group represented by the general formula (7), and L₁' and L₂' may be identical to or different from each other.
In the general formulae (8) and (8'), k and m each represent an integer of 0 to 5, and n represents an integer of 0 or 1 provided that k, m, and n never simultaneously represent 0.

The polycyclic aromatic compound of the present invention is preferably a material for an organic EL device, and is more preferably a host material for an organic EL device.
Specific examples of the polycyclic aromatic compound represented by the general formula (1) of the present invention are shown below. However, the compound is not limited to these exemplified compounds.

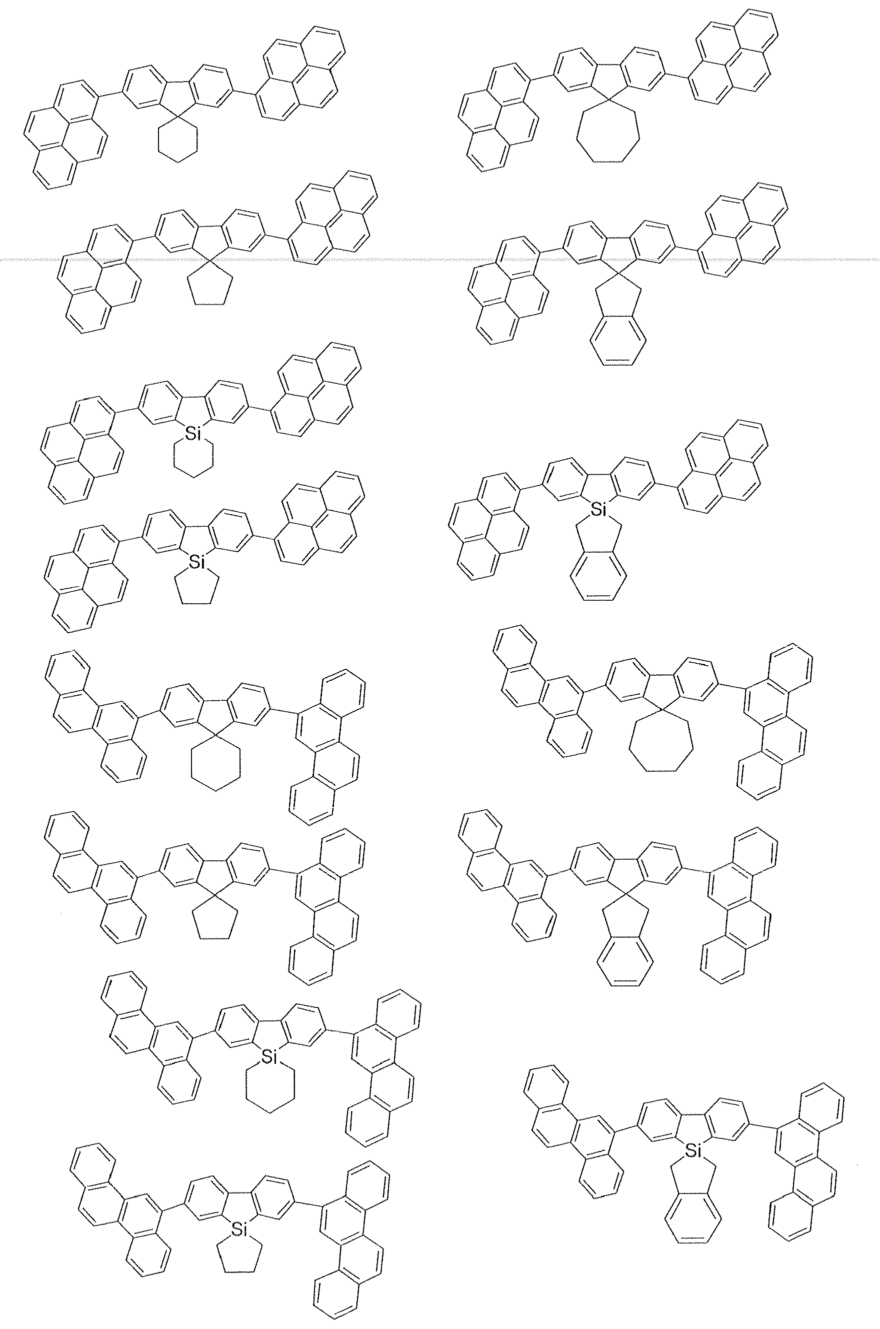

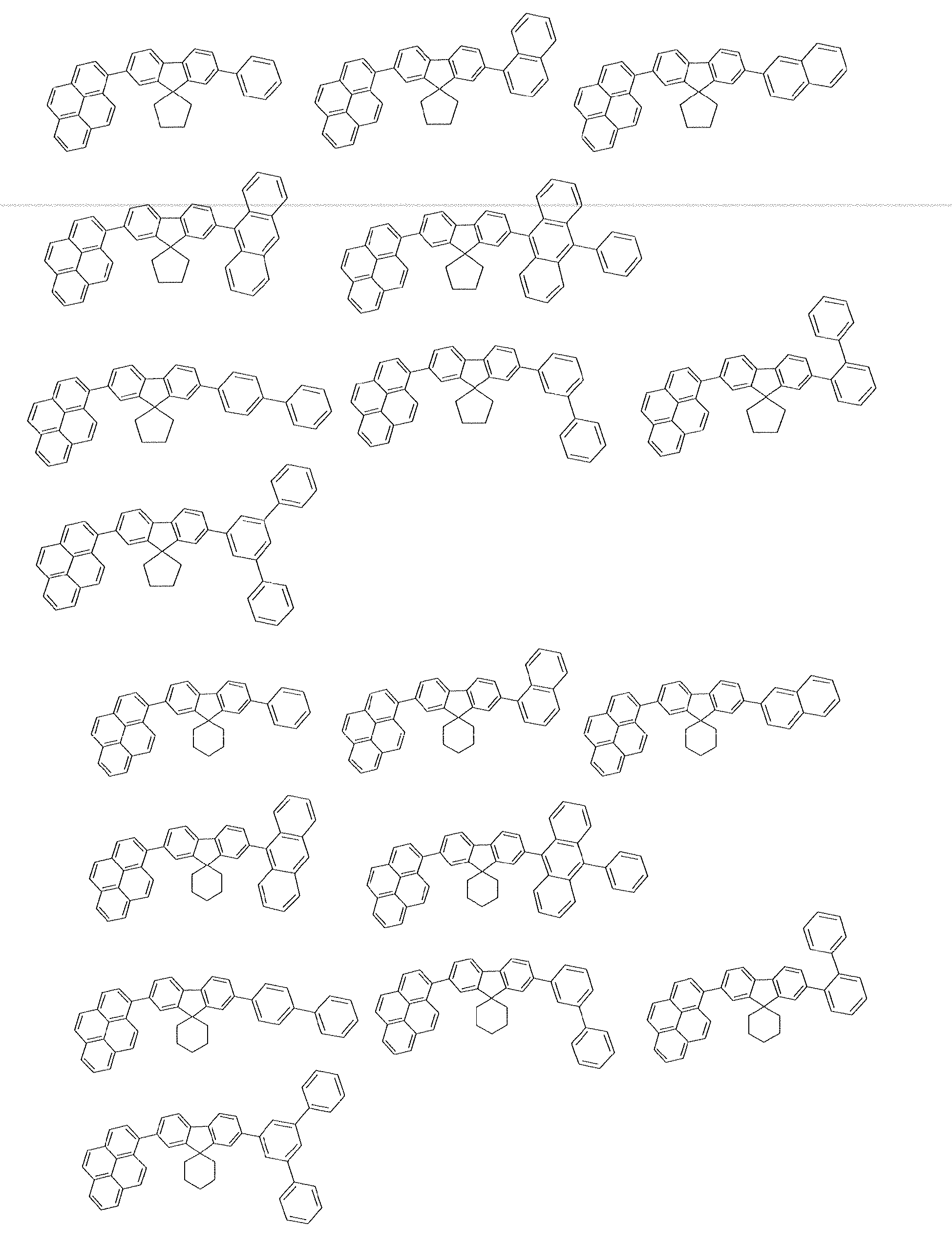

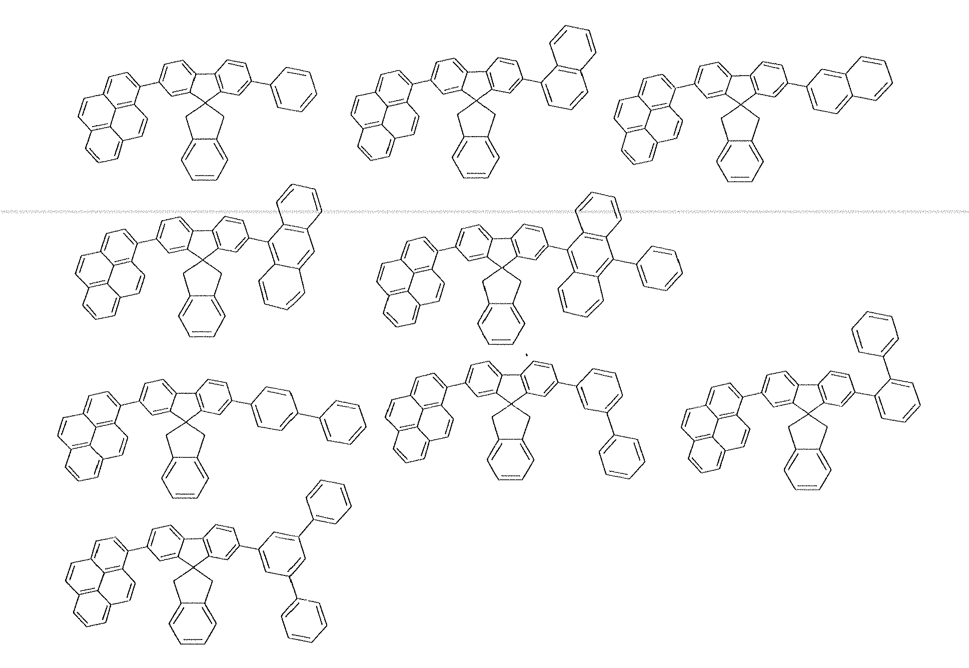

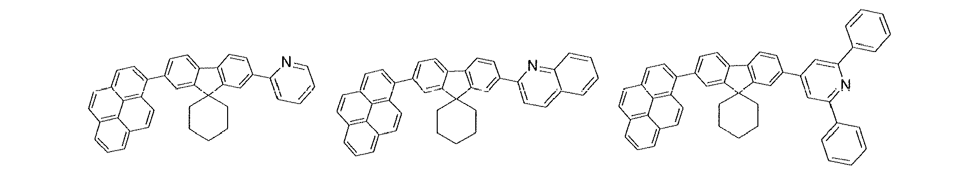

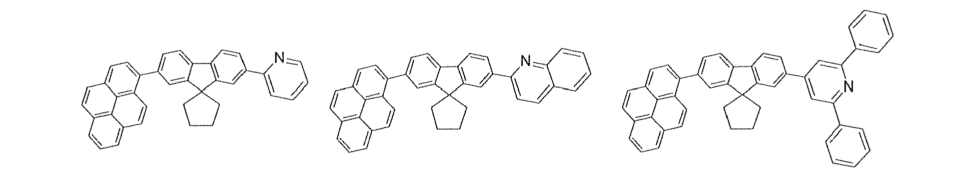

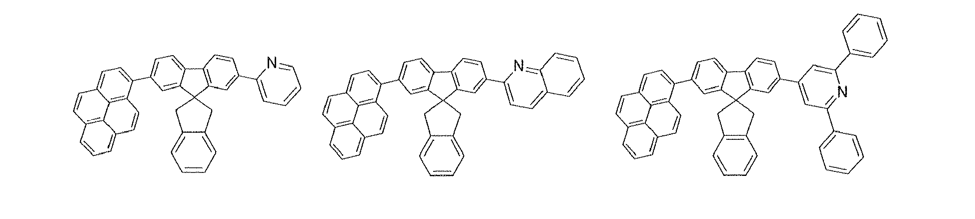

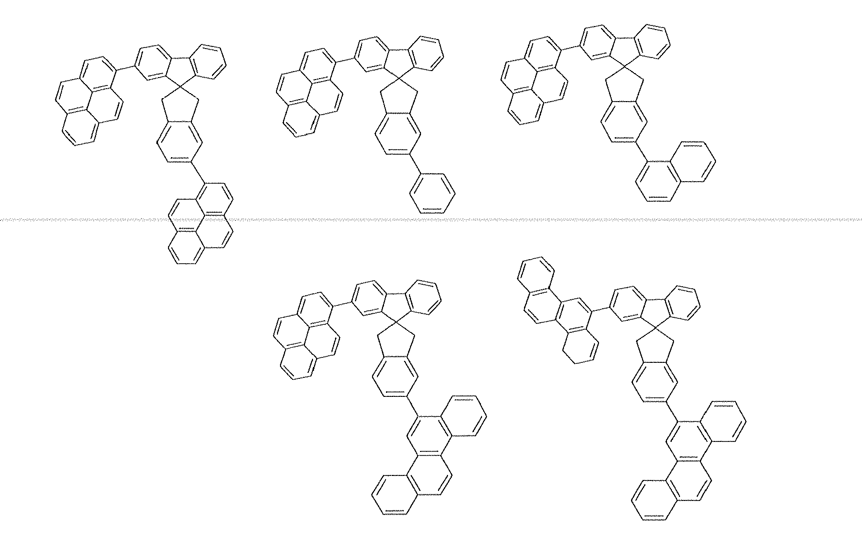

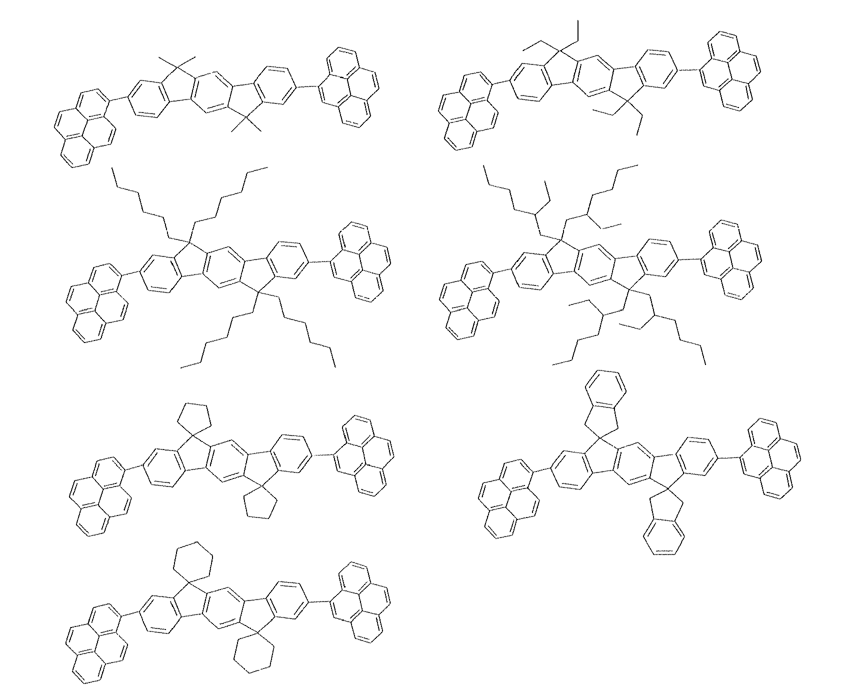

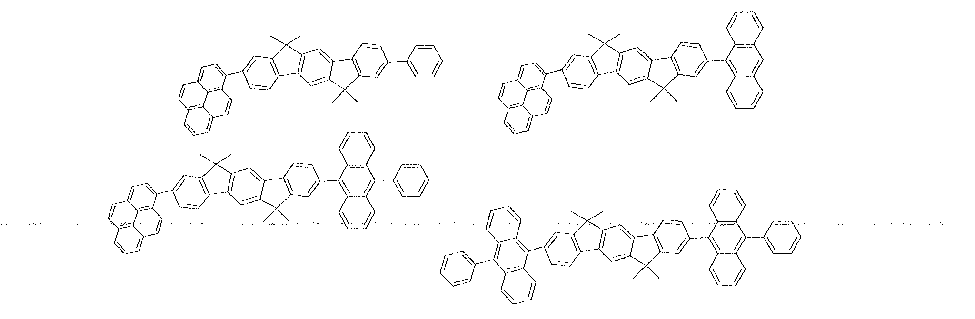

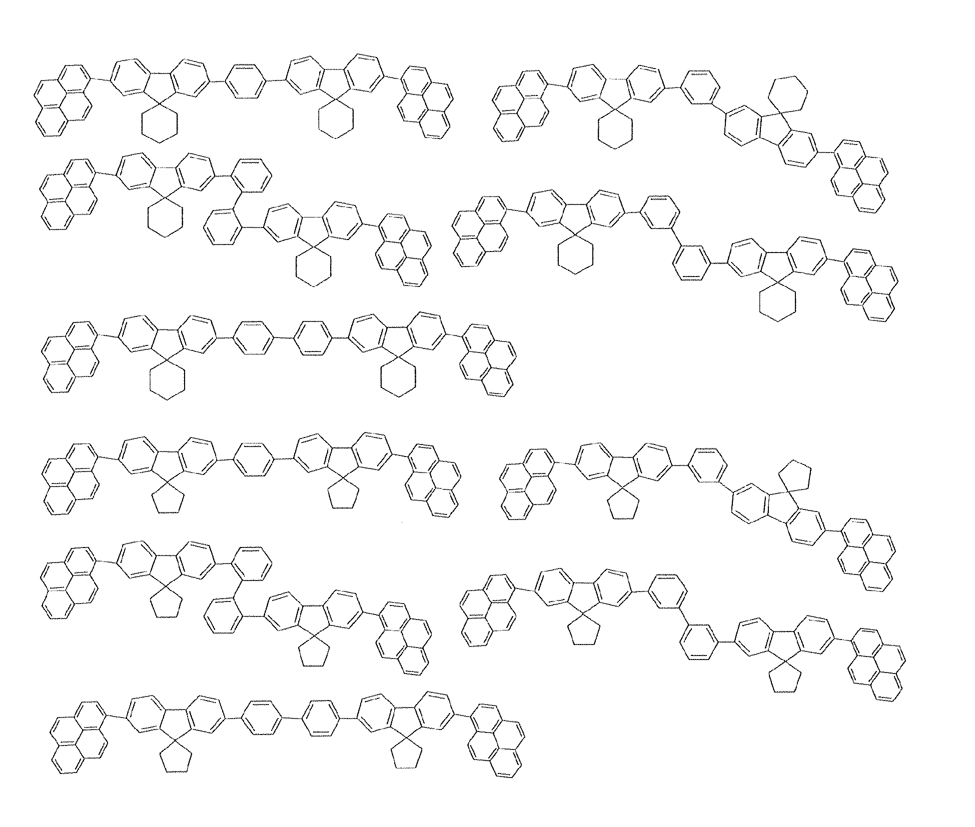

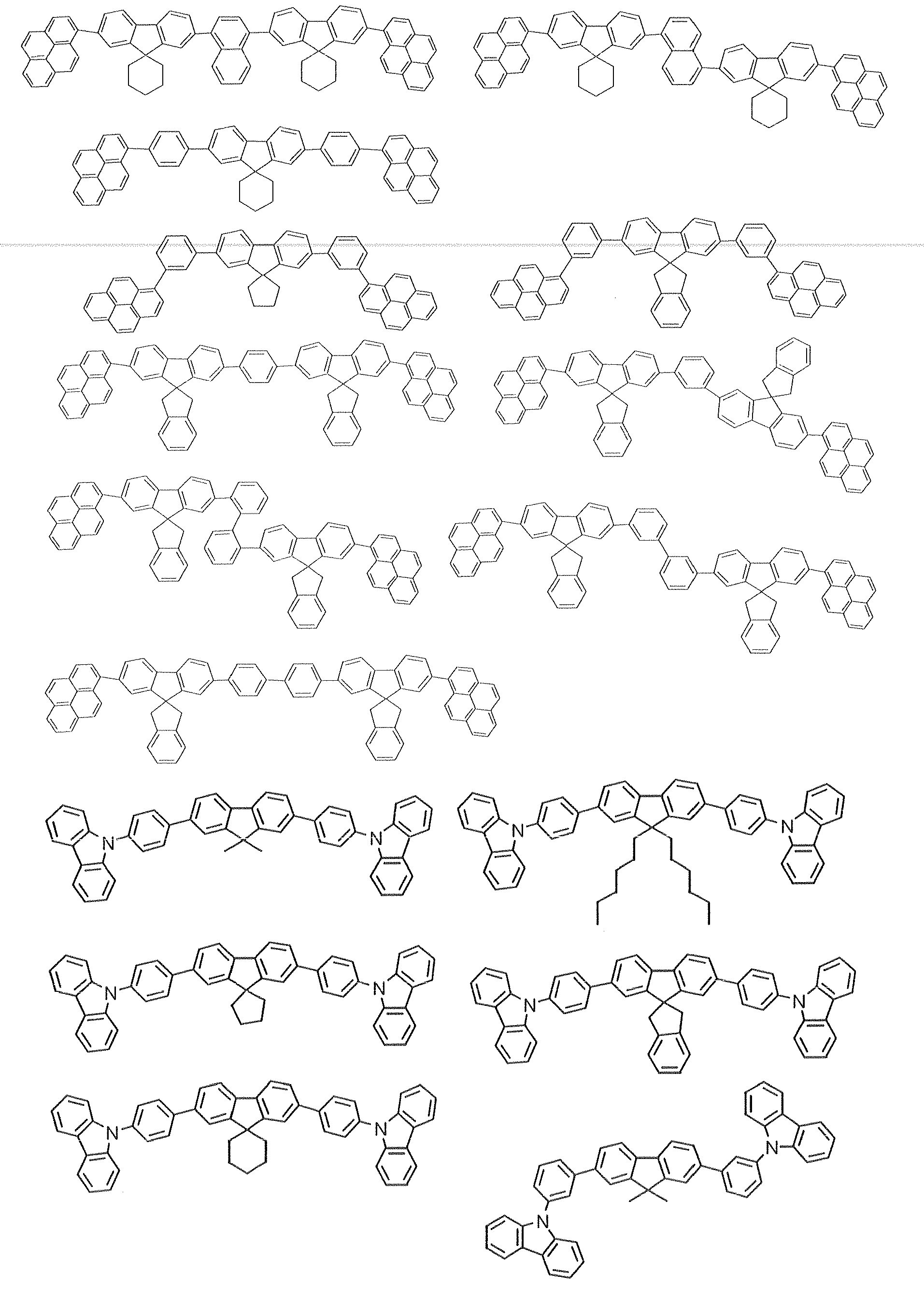

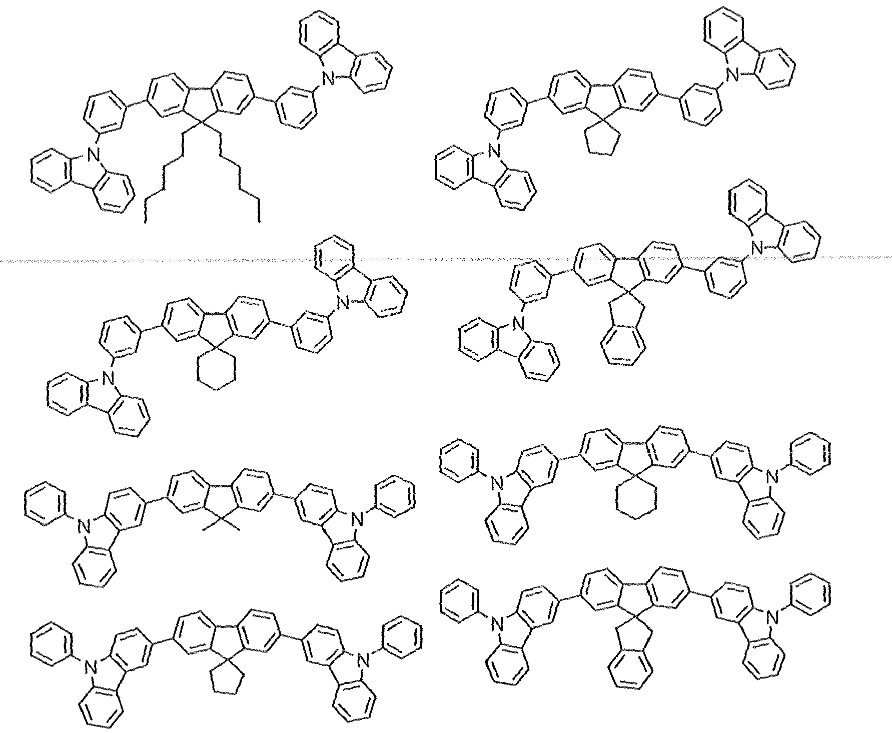

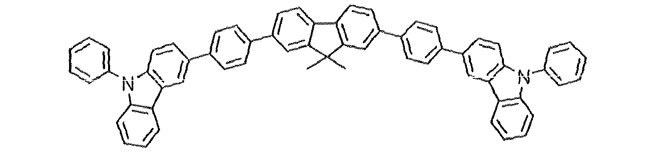

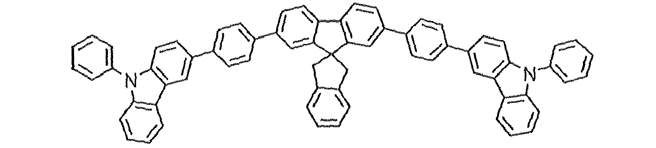

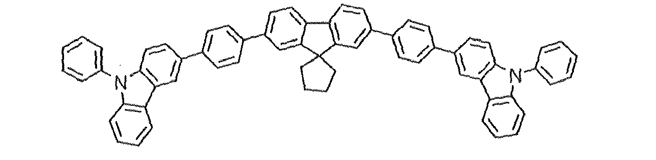

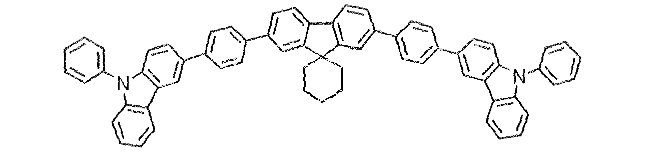

Hereinafter, the device constitution of an organic EL device of the present invention will be described.
The organic EL device of the present invention is an organic electroluminescence device including an organic thin film layer composed of one or multiple layers including at least a light emitting layer, the organic thin film layer being interposed between a cathode and an anode. In the organic electroluminescence device, at least one layer of the organic thin film layer contains the polycyclic aromatic compound alone or as a component of a mixture.
Examples of the representative device constitution of the organic EL device of the present invention may include the following structures, but are not limited thereto:
(1) An anode/light emitting layer/cathode;
(2) An anode/hole injecting layer/light emitting layer/cathode;
(3) An anode/light emitting layer/electron injecting layer/cathode;
(4) An anode/hole injecting layer/light emitting layer/electron injecting layer/cathode;
(5) An anode/organic semiconductor layer/light emitting layer/cathode;
(6) An anode/organic semiconductor layer/electron barrier layer/light emitting layer/cathode;
(7) An anode/organic semiconductor layer/light emitting layer/adhesion improving layer/cathode;
(8) An anode/hole injecting layer/hole transporting layer/light emitting layer/electron injecting layer/cathode;
(9) An anode/insulating layer/light emitting layer/insulating layer/cathode;
(10) An anode /inorganic semiconductor layer/insulating layer/light emitting layer/insulating layer/cathode;
(11) An anode/organic semiconductor layer/insulating layer/light emitting layer/insulating layer/cathode;
(12) An anode/insulating layer/hole injecting layer/hole transporting layer/light emitting layer/ insulating layer/cathode ; and
(13) An anode/insulating layer/hole injecting layer/hole transporting layer/light emitting layer/electron injecting layer/cathode.
   Of those, the constitution (8) is preferably used in general.
   The compound of the present invention may be used in any one of the above-mentioned organic layers, provided that the compound is preferably contained in a light emitting zone or a hole transporting zone in those components. The content of the compound is preferably 30 to 100 mol%.

In the organic EL device of the present invention, the light emitting layer preferably contains the polycyclic aromatic compound represented by the general formula (1), and the content of the compound is preferably 10 to 100 mol%, or more preferably 50 to 99 mol%.
In addition, the light emitting layer may contain the polycyclic aromatic compound represented by the general formula (1) and a fluorescent or phosphorescent dopant.
As the fluorescent dopant, a styrylamine compound represented by the following general formula (9) or an arylamine compound represented by the following general formula (10) can be preferably used.

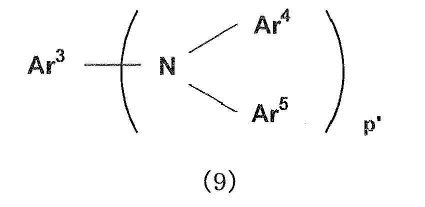

where Ar³ represents a group selected from a phenyl group, a biphenyl group, a terphenyl group, a stilbene group, and a distyrylaryl group, Ar⁴ and Ar⁵ each represent a hydrogen atom or an aromatic group having 6 to 20 carbon atoms, each of Ar³ to Ar⁵ may be substituted, p' represents an integer of 1 to 4, and at least one of Ar⁴ and Ar⁵ is more preferably substituted by a styryl group.

Here, preferable examples of the aromatic group having 6 to 20 carbon atoms include a phenyl group, a naphthyl group, an anthranyl group, a phenanthryl group, and a terphenyl group.

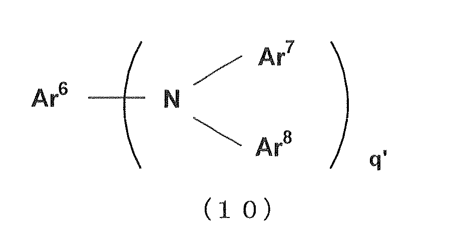

In the general formula (10), Ar⁶ to Ar⁸ each represent an aryl group having 5 to 40 ring carbon atoms, which may be substituted, and q' represents an integer of 1 to 4.
Examples of the aryl group having 5 to 40 ring atoms preferably include phenyl, naphthyl, anthranyl, phenanthryl, pyrenyl, coronyl, biphenyl, terphenyl, pyrrolyl, furanyl, thiophenyl, benzothiophenyl, oxadiazolyl, diphenylanthranyl, indolyl, carbazolyl, pyridyl, benzoquinolyl, fluoranthenyl, acenaphthofluoranthenyl, and stilbene. The aryl group having 5 to 40 ring atoms may be additionally substituted with a substituent. Preferable examples of the substituent include: an alkyl group having 1 to 6 carbon atoms such as an ethyl group, a methyl group, an i-propyl group, an n-propyl group, an s-butyl group, a t-butyl group, a pentyl group, a hexyl group, a cyclopentyl group, or a cyclohexyl group; an alkoxy group having 1 to 6 carbon atoms such as an ethoxy group, a methoxy group, an i-propoxy group, an n-propoxy group, an s-butoxy group, a t-butoxy group, a pentoxy group, a hexyloxy group, a cyclopentoxy group, or a cyclohexyloxy group; an aryl group having 5 to 40 ring atoms; an amino group substituted with the aryl group having 5 to 40 ring atoms; an ester group containing the aryl group having 5 to 40 ring atoms; an ester group containing the alkyl group having 1 to 6 carbon atoms; a cyano group; a nitro group; and a halogen atom such as chlorine, bromine, or iodine.

In addition, the above-mentioned phosphorescent dopant is preferably a metal complex including at least one metal selected from the group consisting of iridium (Ir), ruthenium (Ru), palladium (Pd), platinum (Pt), osmium (Os), and rhenium (Re). A ligand may preferably have at least one skeleton selected from the group consisting of a phenylpyridine skeleton, a bipyridyl skeleton, and a phenanthroline skeleton. Specific examples of such metal complex include, but not limited to, tris(2-phenylpyridine)iridium, tris (2-phenylpyridine) ruthenium, tris(2-phenylpyridine)palladium, bis(2-phenylpyridine)platinum, tris(2-phenylpyridine)osmium, tris(2-phenylpyridine)rhenium, (octaethylporphyrin)platinum, (octaphenylporphyrin)platinum, (actaethylporphyrin)palladium, and (octaphenylporphyrin) palladium. A suitable complex may be selected according to the luminescent color to be required, device performance, and the relationship to a host compound.

The organic EL device of the present invention is prepared on a transparent substrate. The transparent substrate herein is a substrate for supporting the organic EL device, and preferably have a transmittance of light of 50% or more in the visible region of 400 to 700 nm, and is flat and smooth.
Specific examples of the transparent substrate include glass plates and polymer plates. Specific examples of the glass plate include plates made of soda-lime glass, glass containing barium and strontium, leadglass, aluminosilicate glass, borosilicate glass, barium borosilicate glass, and quartz. Specific examples of the polymer plate include plates made of polycarbonate, acrylic resin, polyethylene terephthalate, polyether sulfide, and polysulfone.

The anode in the organic EL device of the present invention has the function of injecting holes into the hole transporting layer or the light emitting layer. It is effective for the anode to have a work function of 4.5 eV or more. Specific examples of the material for the anode to be used in the present invention include indium tin oxide alloys (ITO), tin oxide (NESA), gold, silver, platinum, and copper. Meanwhile, the cathode is preferably made of a material having a small work function, for the purpose of injecting electrons into an electron transporting layer or light emitting layer.
The anode can be prepared by forming a thin film of the electrode materials in accordance with a process such as a vapor deposition process and a sputtering process.
When the light emitted from the light emitting layer is obtained through the anode in the same manner as in the foregoing, the anode preferably has a transmittance of the emitted light of more than 10%. In addition, the anode preferably has a sheet resistivity of several hundredΩ/□ or less. The thickness of the anode is, in general, selected in the range of 10 nm to 1 µm, preferably in the range of 10 to 200 nm depending on the material to be used.

The light emitting layer in the organic EL device of the present invention has the following combined functions:
(i) Injecting function: a function of injecting holes from the anode or the hole injecting layer and injecting electrons from the cathode or the electron injecting layer when an electric field is applied;
(ii) Transporting function: a function of transporting injected charges (i.e., electrons and holes) by the force of the electric field; and
(iii) Light emitting function: a function of providing the field for recombination of electrons and holes to thereby lead to the emission of light.
   However, the easiness of injection may be different between holes and electrons and the ability of transportation expressed by the mobility may be different between holes and electrons. It is preferable that either one of the charges be transferred.
   As the process of forming the light emitting layer, a conventionally known process such as a vapor deposition process, a spin coating process, or an LB process can be used. It is particularly preferable that the light emitting layer be a molecular deposit film.
   The molecular deposit film herein means a thin film formed through deposition of a material compound in a gas phase or a thin film formed through solidification of a material compound in a solution or a liquid phase. In general, the molecular deposit film can be distinguished from the thin film formed in accordance with the LB process (i.e., molecular accumulation film) based on the differences in the aggregation structure and higher order structures and functional differences caused by those structural differences.
   Further, as disclosed in JP-A-57-051781, the light emitting layer can also be formed by dissolving a binder such as a resin and the material compounds into a solvent to prepare a solution, followed by forming a thin film from the prepared solution in accordance with the spin coating process or the like.
   In the present invention, if desired, the light emitting layer may contain other conventionally known light emitting materials except the light emitting material composed of the compound containing a spiro atom of the present invention, or a light emitting layer containing other conventionally known light emitting material may be laminated onto the light emitting layer containing the light emitting material composed of the compound of the present invention as long as the object of the present invention can be attained.

In the organic EL device of the present invention, the hole injecting and transporting layers are layers for helping injection of holes into the light emitting layer and transporting the holes to the light emitting region. The layers each exhibit a great mobility of holes and, in general, have a small ionization energy of 5.5 eV or less. Such the hole injecting and transporting layers are preferably made of a material which transports holes to the light emitting layer with an electric field of a decreased strength. Further, the material preferably has a mobility of holes of at least 10⁻⁴ cm²/V·sec under application of an electric field of 10⁴ to 10⁶ V/cm, for example.
When the compound of the present invention are used in the hole transporting zone, the compounds of the present invention may be used alone or in a mixture with other materials for forming the hole injecting and transporting layers.

The material to be used as a mixture with the compound of the present invention for forming the hole injecting and transporting layers is not particularly limited as long as the material has the above-mentioned desirable properties. The material to be used can be arbitrarily selected from materials which are conventionally used as the charge transporting material of holes in photoconductive materials, and conventionally known materials which are used for the hole injecting layer in organic EL devices. Examples of the materials include an aromatic tertiary amine, a hydrazone derivative, a carbazole derivative, a triazole derivative, an imidazole derivative, polyvinylcarbazole, and polyethylenedioxythiophene/poly sulfonic acid (PEDOT/PSS). Further, specific examples of the materials include: a triazole derivative (see, for example, US 3,112,197); an oxadiazole derivative (see, for example, US 3, 189, 447) ; an imidazole derivative (see, for example, JP-B-37-016096); a polyarylalkane derivative (see, for example, US 3,615,402, US 3,820,989, US 3,542,544, JP-B-45-000555, JP-B-51-010983, JP-A-51-093224, JP-A-55-017105, JP-A-56-004148, JP-A-55-108667, JP-A-55-156953, and JP-A-56-036656) ; a pyrazoline derivative and a pyrazolane derivative (see, for example, US 3,180,729, US 4, 278, 746, JP-A-55-088064, JP-A-55-088065, JP-A-49-105537, JP-A-55-051086, JP-A-56-080051, JP-A-56-088141, JP-A-57-045545, JP-A-54-112637, and JP-A-55-074546); a phenylenediamine derivative (see, for example, US 3,615,404, JP-B-51-010105, JP-B-46-003712, JP-B-47-025336, JP-A-54-053435, JP-A-54-110536, and JP-A-54-119925) ; an arylamine derivative (see, for example, US 3,567,450, US 3,180,703, US 3,240,597, US 3, 658, 520, US 4, 232, 103, US 4,175,961, US 4, 012, 376, JP-B-49-035702, JP-B-39-027577, JP-A-55-144250, JP-A-56-119132, JP-A-56-022437, and DE 1,110,518); an amino-substituted chalcone derivative (see, for example, US 3, 526, 501) ; an oxazole derivative (those disclosed in US 3,257,203 and the like); a styrylanthracene derivative (see, for example, JP-A-56-046234); a fluorenone derivative (see, for example, JP-A-54-110837); a hydrazone derivative (see, for example, US 3,717,462, JP-A-54-059143, JP-A-55-052063, JP-A-55-052064, JP-A-55-046760, JP-A-55-085495, JP-A-57-011350, JP-A-57-148749, and JP-A-02-311591); a stilbene derivative (see, for example, JP-A-61-210363, JP-A-61-228451, JP-A-61-014642, JP-A-61-072255, JP-A-62-047646, JP-A-62-036674, JP-A-62-010652, JP-A-62-030255, JP-A-60-093455, JP-A-60-094462, JP-A-60-174749, and JP-A-60-175052); a silazane derivative (US 4,950,950); a polysilane-based copolymer (JP-A-02-204996); an aniline-based copolymer (JP-A-02-282263); and an conductive high molecular weight oligomer (particularly a thiophene oligomer) disclosed in JP-A-01-211399.

Any one of the above-mentioned materials can be used as the material for the hole injecting layer. A porphyrin compound, an aromatic tertiary amine compound, and a styrylamine compound (see, for example, US 4,127,412, JP-A-53-027033, JP-A-54-058445, JP-A-54-149634, JP-A-54-064299, JP-A-55-079450, JP-A-55-144250, JP-A-56-119132, JP-A-61-295558, JP-A-61-098353, and JP-A-63-295695 are preferably used, and the aromatic tertiary amine compound is particularly preferably used.
Further examples of the material include compounds having two fused aromatic rings in the molecule such as 4,4'-bis(N-(1-naphthyl)-N-phenylamino)biphenyl (hereinafter abbreviated as NPD) as disclosed in US 5,061,569, and a compound in which three triphenylamine units are bonded together in a star-burst shape such as 4,4',4"-tris(N-(3-methylphenyl)-N-phenylamino)triphenylamine (hereinafter abbreviated as MTDATA) as disclosed in JP-A-04-308688.
Further, inorganic compounds such as Si of the p-type and SiC of the p-type can also be used as the material for the hole injecting layer in addition to the aromatic dimethylidene-based compounds described above as the material for the light emitting layer.
The hole injecting and transporting layers can be formed by producing thin films of the compound described above in accordance with a conventionally known process such as the vacuum deposition process, the spin coating process, the casting process, and the LB process. The thickness of each of the hole injecting and transporting layers is not particularly limited. In general, the thickness thereof is 5 nm to 5 µm. The hole injecting and transporting layers may preferably contain the compound of the present invention in the hole transporting zone, and may be composed of a single layer made of one or more materials described above, or may be a laminate of hole injecting and transporting layers composed of compounds different from those of the above-mentioned hole injecting and transporting layers.

In the organic EL device of the present invention, an organic semiconductor layer helps the injection of holes or electrons into the light emitting layer. The organic semiconductor layer preferably has a conductivity of 10⁻¹⁰ S/cm or more. As the material for such the organic semiconductor layer, oligomers containing thiophene, conductive oligomers such as oligomers containing arylamine and conductive dendrimers such as dendrimers containing arylamine which are disclosed in JP-A-08-193191, and the like can be used.

In the organic EL device of the present invention, the electron injecting layer helps the injection of electrons into the light emitting layer, and exhibits a great mobility of electrons. The adhesion improving layer is a layer made of a material which particularly exhibits improved adhesion with the cathode in the electron injecting layers. As the material used for the electron injecting layer, 8-hydroxyquinoline, metal complexes of derivatives thereof, and oxadiazole derivatives are preferable.
A specific example of the metal complex of 8-hydroxyquinoline or the metal complexes of the derivatives thereof includes metal chelate oxinoid compounds containing the chelate of oxine (in general, 8-quinolinol or 8-hydroxyquinoline). For example, tris (8-quinolinolato) aluminum (Alq) may be used as materials for an electron injecting layer.
On the other hand, examples of the oxadiazole derivative include electron transfer compounds each represented by the following general formulae:

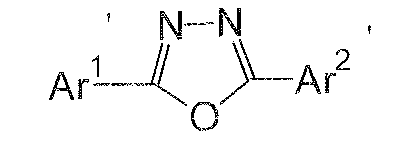

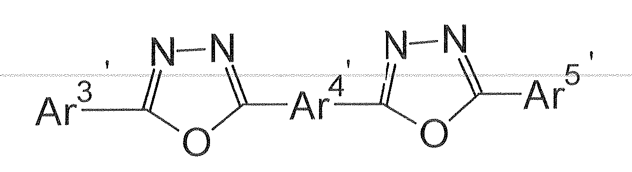

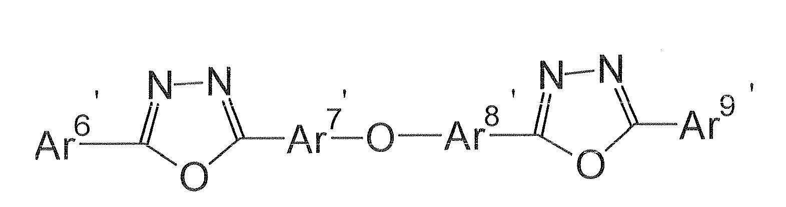

where, Ar^{1'}, Ar^{2'}, Ar^{3'}, Ar^{5'}, Ar^{6'}, and Ar^{9'} each represent a substituted or unsubstituted aryl group and may represent the same group or different groups. Ar^{4'}, Ar^{7'}, and Ar^{8'} each represent a substituted or unsubstituted arylene group and may represent the same group or different groups.
Examples of the aryl group include a phenyl group, a biphenyl group, an anthranyl group, a perylenyl group, and a pyrenyl group. Examples of the arylene group include a phenylene group, a naphthylene group, a biphenylene group, an anthranylene group, a perylenylene group, and a pyrenylene group. Examples of the substituent include an alkyl group having 1 to 10 carbon atoms, an alkoxy group having 1 to 10 carbon atoms, and a cyano group. As the electron transfer compound, compounds which can form thin films are preferable.

Specific examples of the electron transfer compounds include the following.

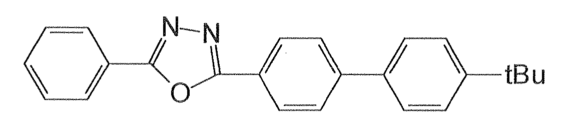

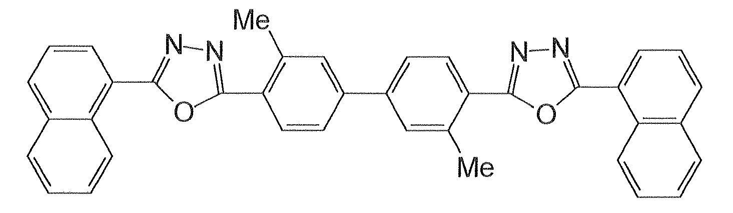

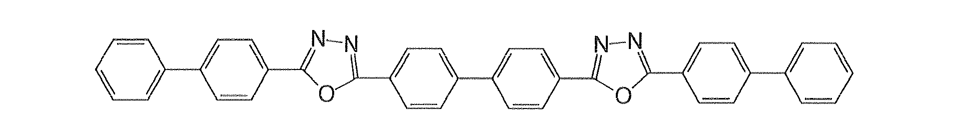

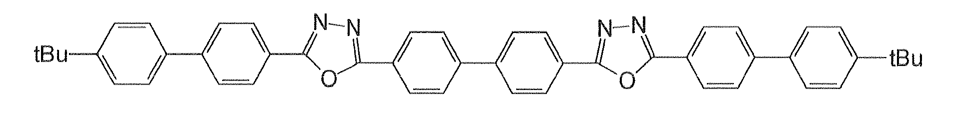

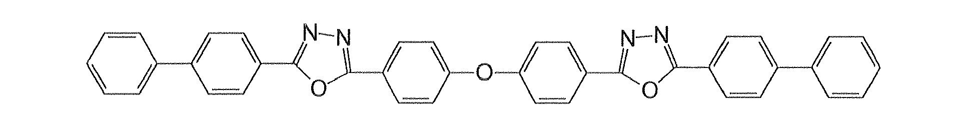

A preferable embodiment of the organic EL device of the present invention includes a device containing a reducing dopant in the region of electron transport or in the interfacial region of the cathode and the organic layer. Here, the reducing dopant is defined as a substance which can reduce a compound having the electron transporting property. Accordingly, various compounds can be used as the reducing dopant as long as the compounds have a certain reductive property. For example, at least one substance selected from the group consisting of alkali metals, alkaline earth metals, rare earth metals, alkali metal oxides, alkali metal halides, alkaline earth metal oxides, alkaline earth metal halides, rare earth metal oxides or rare earth metal halides, organic complexes of alkali metals, organic complexes of alkaline earth metals, and organic complexes of rare earth metals can be suitably used.
To be more specific, preferable examples of the reducing dopant include at least one alkali metal selected from the group consisting of Na (work function: 2.36 eV), K (work function: 2.28 eV), Rb (work function: 2.16 eV), and Cs (work function: 1. 95 eV), and at least one alkaline earth metal selected from the group consisting of Ca (work function: 2.9 eV), Sr (work function: 2.0 to 2.5 eV), and Ba (work function: 2. 52 eV), and substances having a work function of 2.9 eV or less are particulary preferable.
Of those, at least one alkali metal selected from the group consisting of K, Rb, and Cs is more preferable, Rb and Cs are still more preferable, and Cs is most preferable as the reducing dopant. Those alkali metals have great reducing ability in particular, and the luminance of the light emission can be increased and the lifetime of the organic EL device can be prolonged by addition of a relatively small amount of the alkali metal into the electron injecting zone. As the reducing dopant having a work function of 2.9 eV or less, combinations of two or more alkali metals thereof are also preferable. Combinations including Cs such as the combinations of: Cs and Na; Cs and K; Cs and Rb; and Cs, Na, and K are particularly preferable. The reducing ability can be efficiently exerted when the reducing dopant contains the combination including Cs. The luminance of the light emission can be increased and the lifetime of the organic EL device can be prolonged by adding the combination including Cs into the electron injecting zone.

In the organic EL device of the present invention, an electron injecting layer which is constituted of an insulating material or a semiconductor may be additionally provided between the cathode and the organic layer. Leak of electric current can be effectively prevented by the electron injecting layer, to thereby improve the electron injecting property.
As such the insulating material, at least one metal compound selected from the group consisting of alkali metal chalcogenides, alkaline earth metal chalcogenides, alkali metal halides, and alkaline earth metal halides is preferably used. It is preferable that the electron injecting layer be constituted of the alkali metal chalcogenide and the like since the electron injecting property can be further improved. Specific examples of the alkali metal chalcogenide preferably include Li₂O, K₂O, Na₂S, Na₂Se, and Na₂O. Preferable examples of the alkaline earth metal chalcogenide include CaO, BaO, SrO, BeO, BaS, and CaSe. Preferable examples of the alkali metal halide include LiF, NaF, KF, LiCl, KCl, and NaCl. Preferable examples of the alkaline earth metal halide include fluorides such asCaF₂, BaF₂, SrF₂, MgF₂, and BeF₂ and halides other than the fluorides.

Examples of the semiconductor include one or a combination of two or more oxides, nitrides, or oxide nitrides having at least one element selected from Ba, Ca, Sr, Yb, Al, Ga, In, Li, Na, Cd, Mg, Si, Ta, Sb, and Zn. It is preferable that the inorganic compound constituting the electron transporting layer forms a crystallite or amorphous insulating thin film. When the electron transporting layer is constituted of those insulating thin films, a more uniform thin film can be formed, whereby defects in pixels such as dark spots can be decreased. Examples of the inorganic compound include alkali metal chalcogenides, alkaline earth metal chalcogenides, alkali metal halides, and alkaline earth metal halides, all of which are described above.

As the cathode of the organic EL device of the present invention, a material such as a metal, an alloy, a conductive compound, or a mixture of those materials having a small work function (i.e., 4 eV or less) is used as the electrode material. Specific examples of the electrode material include sodium, sodium-potassium alloys, magnesium, lithium, magnesium-silver alloys, aluminum/aluminum oxide, aluminum-lithium alloys, indium, and rare earth metals.
The cathode can be prepared by forming a thin film of those electrode materials in accordance with a process such as the vapor deposition process and the sputtering process. When the light emitted from the light emitting layer is obtained through the cathode, the cathode preferably has a transmittance of the emitted light of more than 10%. In addition, the cathode preferably has the sheet resistivity of several hundred Ω/□ or less. The thickness of the cathode is, in general, selected in the range of 10 nm to 1 µm, and preferably in the range of 50 to 200 nm.

Defects in pixels tend to generate in organic EL device of the present invention since leak and short circuit occur when an electric field is applied to ultra-thin films. To prevent the generation of the defects, a layer of a thin film having an insulating property is preferably inserted between the pair of electrodes.
Examples of the material to be used for the insulating layer include aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide, and vanadium oxide. Mixtures and laminates of those compounds may also be used.
The organic EL device can be prepared by forming the anode, the light emitting layer, and if needed, the hole injecting layer and the electron injecting layer in accordance with the illustrated materials and process, and by additionally forming the cathode. The organic EL device may also be prepared by forming the above-mentioned layers in the order reverse to the stated order, i.e., the cathode being formed in the first step and the anode in the last step.
Hereinafter, an embodiment of the process for preparing an organic EL device having a construction in which an anode, a hole injecting layer, a light emitting layer, an electron injecting layer, and a cathode are disposed sequentially on a transparent substrate will be described.

First, on a transparent substrate, a thin film made of a material for the anode is formed in accordance with the vapor deposition, process, the sputtering process, or the like so that the thickness of the formed thin film is 1 µm or less, preferably in the range of 10 to 200 nm, to thereby provide the anode. Then, when a hole injecting layer is formed on the anode, the hole injecting layer can be formed in accordance with the vacuum deposition process, the spin coating process, the casting process, the LB process, or the like as described above. The vacuum deposition process is preferable since a uniform film can be easily obtained and the possibility of pin holes being formed is small. When the hole injecting layer is formed in accordance with the vacuum deposition process, it is preferable, in general, that the conditions be suitably selected from the following ranges: the temperature of the source of the deposition of 50 to 450°C; the vacuum of 10⁻⁷ to 10⁻³ Torr; the rate of deposition of 0.01 to 50 nm/second; the temperature of the substrate of -50 to 300°C; and the thickness of the film of 5 nm to 5 µm, although the conditions of the vacuum deposition vary depending on the compound to be used (i.e., the material for the hole injecting layer) and the crystal structure and the recombination structure of the hole injecting layer to be formed.
Then, the light emitting layer is formed on the hole injecting layer. By using the desired organic light emitting material, a thin film of the organic light emitting material can be formed in accordance with the vacuum deposition process, the sputtering process, the spin coating process, the casting process, or the like, and the formed thin film is used as the light emitting layer. The vacuum deposition process is preferable since a uniform film can be easily obtained and the possibility of pin holes being formed is small. When the light emitting layer is formed in accordance with the vacuum deposition process, in general, the conditions of the vacuum deposition can be selected from the same ranges as those of the hole injecting layer although the conditions vary depending on the compound to be used.

Next, when an electron injecting layer is formed on the light emitting layer, it is preferable that the electron injecting layer be formed in accordance with the vacuum deposition process since a uniform film must be obtained like the hole injecting layer and the light emitting layer. The conditions of the vacuum deposition can be selected from the same ranges as those of the hole injecting layer and the light emitting layer.
When the vacuum deposition process is used, the polycyclic aromatic compounds of the present invention can be deposited from vapor in combination with other materials although the situation varies depending on which layer in the light emitting zone or the hole transporting zone is to contain the compounds. When the spin coating process is used, the compounds can be incorporated into the layer by mixing the compounds with other materials.
Finally, a cathode is laminated on the electron injecting layer, whereby an organic EL device can be obtained.
The cathode is made of metal and can be formed in accordance with the vacuum deposition process, the sputtering process, or the like. The vacuum deposition process is preferably used in order to prevent the lower organic layers from being damaged during the formation of the film.
In the above-mentioned preparation of the organic EL device, it is preferable that the layers be formed successively from the anode to the cathode after being evacuated once.

Note that the process for forming each layer in the organic EL device of the present invention is not particularly limited. A conventionally known process such as the vacuum deposition process and the spin coating process can be used as the forming process. The organic thin film layer which is used in the organic EL device of the present invention and contains the polycyclic aromatic compounds represented by the general formula (1) can be formed in accordance with a conventionally known process such as the vacuum deposition process and the molecular beam epitaxy process (i.e., MBE process), or in accordance with a coating process such as the dipping process, the spin coating process, the casting process, the bar coating process, and the roll coating process by using a solution prepared by dissolving the compounds into a solvent.

A material for forming a luminescent coating film of the present invention is composed of an organic solvent solution containing the polycyclic aromatic compound. The term "material for forming a luminescent coating film" refers to a material of which a coating film is formed to produce, in, for example, an organic EL device, an organic compound layer involved in light emission, specifically a light emitting layer, a hole injecting (transporting) layer, an electron injecting (transporting) layer, or the like.

In the case where the organic EL device of the present invention is produced by using the material for forming a luminescent coating film of the present invention or any other material for forming a coating film according to an application method, examples of an organic solvent used for dissolving the polycyclic aromatic compound of the present invention include: halogen-based hydrocarbon-based solvents such as dichloromethane, dichloroethane, chloroform, carbon tetrachloride, tetrachloroethane, trichloroethane, chlorobenzene, dichlorobenzene, and chlorotoluene; ether-based solvents such as dibutyl ether, tetrahydrofuran, dioxane, and anisole; alcohol-based solvents such as methanol, ethanol, propanol, butanol, pentanol, hexanol, cyclohexanol, methyl cellosolve, ethyl cellosolve, and ethylene glycol; hydrocarbon-based solvents such as benzene, toluene, xylene, ethylbenzene, hexane, octane, and decane; and ester-based solvents such as ethyl acetate, butyl acetate, and amyl acetate. Of those, halogen-based hydrocarbon-based solvents, hydrocarbon-based solvents, and ether-based solvents are preferable. In addition, one kind of those solvents may be used alone, or two or more kinds of them may be used in combination. It should be noted that a solvent that can be used is not limited to those solvents.
In addition, a dopant may be dissolved in the solution of the material for forming a luminescent coating film of the present invention in advance as desired. An amine compound represented by the general formula (9) or (10) can be used as the dopant. In addition, any one of other various additives may be dissolved in the solution as required.

The thickness of each organic layer which forms the organic thin film layer of the organic EL device of the present invention is not particularly limited. In general, an excessively thin layer tends to cause defects such as pin holes, whereas an excessively thick layer requires high applied voltage to thereby lower the efficiency. Therefore, in general, a thickness in the range of several nm to 1 µm is preferable.
It should be noted that when a direct voltage is applied to the organic EL device, the light emission can be observed by applying the voltage of 5 to 40 V in the condition where the anode is connected to a positive electrode (+) and the cathode is connected to a negative electrode (--) . When a voltage is applied to the organic EL device in the condition where the connection is reversed, no electric current is observed and no light is emitted at all. When alternating voltage is applied to the organic EL device, uniform light emission is observed only when the anode is a positive electrode (+) and the cathode is a negative electrode (-). The alternating voltage being applied may have an arbitrary type of wave shape.

### EXAMPLES

Next, the present invention will be described in more detail by way of examples. However, the present invention is not limited to the following examples without departing from its gist. Synthesis Example 1 (synthesis of spiro[cyclohexane-1,9'-fluorene-2',7'-bis(1-pyrenyl)] (Compound 1))
Compound 1 was synthesized as described below.

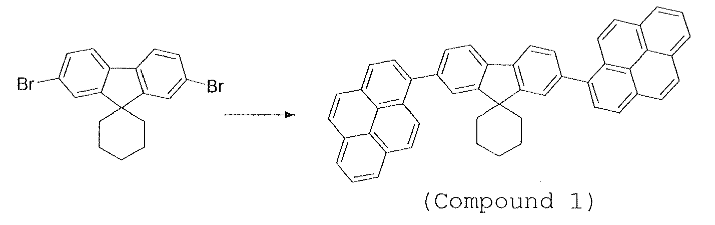

Under an argon atmosphere, spiro[cyclohexane-1,9'-fluorene-2',7'-dibromide] (2.0 g, 5.1 mmol), pyrene-1-boronic acid (3.0 g, 12 mmol), tetrakis(triphenylphosphine)palladium (0.35 g, 0.3 mmol), toluene (20 mL), and a 2-M aqueous solution of sodium carbonate (15 mL, 30 mmol) were added to a 200-mL three-necked flask, and the whole was heated at 80°C for 9 hours. Water (100 mL) was added to the reaction liquid in such a manner that a solid would be precipitated. Then, the solid was filtered. The resultant was purified by means of silica gel column chromatography (amount 1.75 g, yield 54 %). The purified product was identified as Compound 1 on the basis of ¹H-NMR and a field desorption mass spectrum (FD-MS). The measured value of the FD-MS was 634.

### Synthesis Example 2 (synthesis of spiro[5-(1-pyrenyl)indan-2,9'-fluorene-2'-(1-pyrenyl)] (Compound 2))

Compound 2 was synthesized as described below.

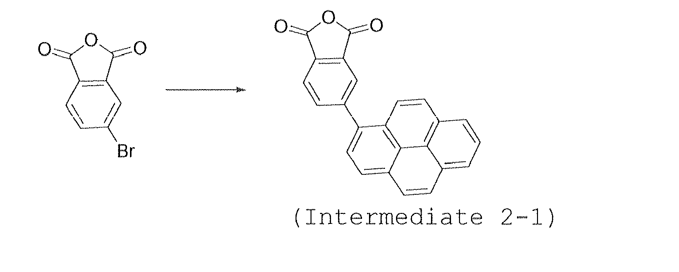

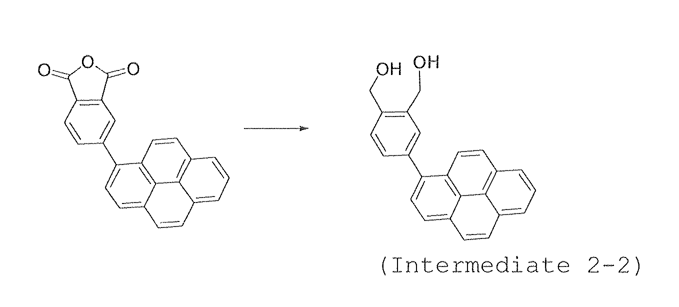

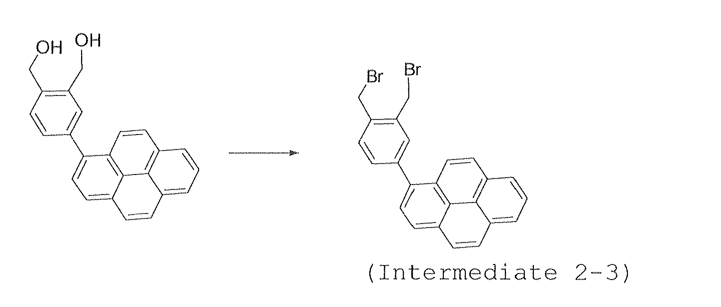

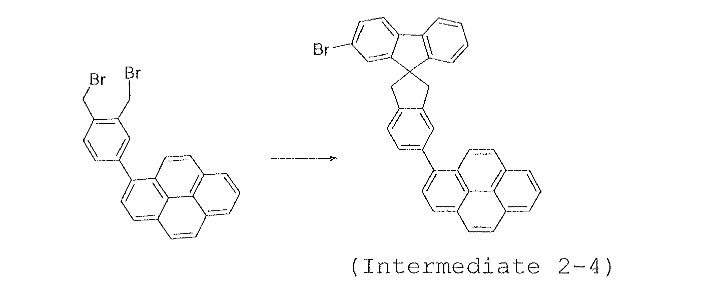

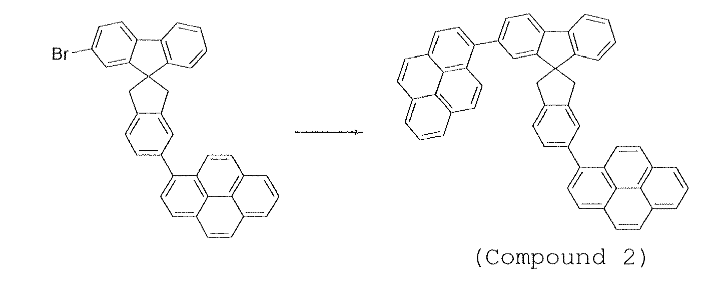

### (1) Synthesis of Intermediate 2-1

Under an argon atmosphere, 4-bromophthalic anhydride (3.3 g, 12 mmol), pyrene-1-boronic acid (3.0 g, 12 mmol), tetrakis(triphenylphosphine)palladium (0.35 g, 0.3 mmol), toluene (20 mL), and a 2-M aqueous solution of sodium carbonate (18 mL, 36 mmol) were added to a 200-mL three-necked flask, and the whole was heated at 80°C for 9 hours. Water (100 mL) was added to the reaction liquid in such a manner that a solid would be precipitated. Then, the solid was filtered. The resultant was purified by means of silica gel column chromatography, whereby Intermediate 2-1 was obtained (amount 2.5 g, yield 61 %).

### (2) Synthesis of Intermediate 2-2

Under an argon atmosphere, lithium aluminum hydride (0.55 g, 15 mmol) was suspended into anhydrous tetrahydrofuran (50 mL). A solution of Intermediate 2-1 (2.5 g, 7.2 mmol) in anhydrous tetrahydrofuran (120 ml) was slowly dropped to the suspension at room temperature, and the whole was stirred for 7 hours. After ethyl acetate, water, and 1N hydrochloric acid had been added to the reaction liquid, an organic layer was separated and washed with a saturated brine. The resultant was dried with anhydrous magnesium sulfate, and was then concentrated under reduced pressure by using a rotary evaporator, thereby resulting in Intermediate 2-2 as a white solid (amount 1.7 g, yield 70 %).

### (3) Synthesis of Intermediate 2-3

Intermediate 2-2 (1.7g, 5.0 mmol) was dissolved in methylene chloride (100 mL). Triphenylphosphine (3.3 g, 13 mmol) and N-bromosuccinimide (2.3 g, 13.2 mmol) were added to the solution, and the whole was stirred at room temperature for 3 hours. A saturated aqueous solution of sodium hydrogen carbonate was added to the reaction liquid in such a manner that an organic layer would be separated. The organic layer was washed with a saturated brine. The organic layer was dried with magnesium sulfate, and was then concentrated under reduced pressure by using a rotary evaporator, whereby a solid was obtained. The solid was purified by means of silica gel column chromatography (eluting solvent; methylene chloride), thereby resulting in Intermediate 2-3 as a white solid (amount 2.0 g, yield 86 %).

### (4) Synthesis of Intermediate 2-4

2-bromofluorene (1.1 g, 4.3 mmol), Intermediate 2-3 (2.0 g, 4 . 3 mmol), and benzyltriethylammonium chloride (0.02 g, 0.08 mmol) were dissolved in toluene (5 mL) and dimethyl sulfoxide (0.2 mL) . A 50 wt% aqueous solution of NaOH (1.2 g) was added to the solution, and the whole was stirred at 80°C for 2 days. Water (100 mL) and toluene (100 mL) were added to the reaction liquid in such a manner that an organic layer would be separated. The organic layer was washed with a saturated brine, and was then dried with anhydrous magnesium sulfate. The resultant was concentrated under reduced pressure by using a rotary evaporator, and then the residue was purified by means of silica gel column chromatography (eluting solvent; methylene chloride), thereby resulting in Intermediate 2-4 as a white solid (amount 1.3 g, yield 58 %).

### (5) Synthesis of spiro[5-(1-pyrenyl)indan-2,9'-fluorene-2'-(1-pyrenyl)]

### (Compound 2)

Under an argon atmosphere, Intermediate 2-4 (1.3 g, 2.4 mmol), pyrene-1-boronic acid (0.74 g, 3.0 mmol), tetrakis(triphenylphosphine)palladium (0.1 g, 0.1 mmol), toluene (20 mL), and a 2-M aqueous solution of sodium carbonate (3.6 mL, 7.2 mmol) were added to a 200-mL three-necked flask, and the whole was heated at 80°C for 9 hours. Water (100 mL) was added to the reaction liquid in such a manner that a solid would be precipitated. Then, the solid was filtered. The resultant was purified by means of silica gel column chromatography (amount 1.3 g, yield 81 %). The purified product was identified as Compound 2 on the basis of ¹H-NMR and FD-MS. The measured value of the FD-MS was 668.

### Synthesis Example 3 (synthesis of Compound 3)

Compound 3 was synthesized as described below.

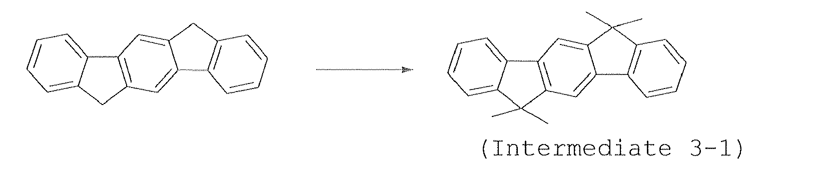

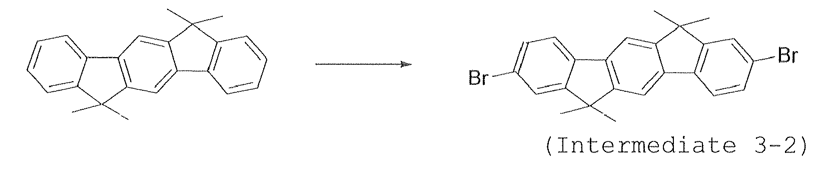

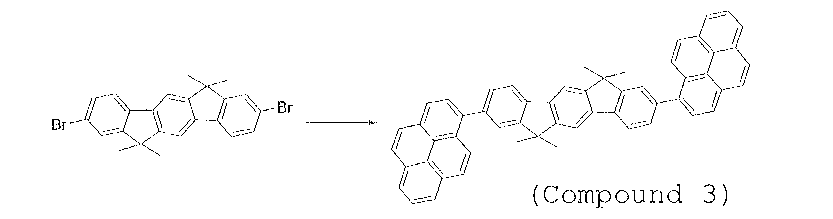

### (1) Synthesis of Intermediate 3-1

6,12-dihydroindeno[1,2-b]fluorene (the synthesis method of which was described in J. Org. Chem., 56, 3, 1991, 1210-1217) (2.0 g, 7.9 mmol), benzyltriethylammonium chloride (0.09 g, 0.4 mmol), dimethyl sulfoxide (0.2 mL), and a 50 wt% aqueous solution of NaOH (5 g) were added to a flask. Next, iodomethane (5.6 g, 39 mmol) was dropped to the mixture, and the whole was stirred for 2 hours. Water (100 mL) and toluene (100 mL) were added to the reaction liquid in such a manner that an organic layer would be separated. The organic layer was washed with a saturated brine, and was then dried with anhydrous magnesium sulfate. The resultant was concentrated under reduced pressure by using a rotary evaporator, and then the residue was purified by means of silica gel column chromatography, whereby Intermediate 3-1 was obtained (amount 2.1 g, yield 85 %).

### (2) Synthesis of Intermediate 3-2

Intermediate 3-1 (2.1 g, 6.7 mmol) and iron (III) chloride were added to anhydrous methylene chloride (10 mL) . Then, bromine (2. 7 g, 17 mmol) was slowly dropped to the solution while the solution was stirred. After having been stirred at room temperature for 24 hours, the reaction liquid was washed with a saturated aqueous solution of sodium hydrogen carbonate and water, and an organic layer was dried with anhydrous sodium sulfate. The resultant was concentrated under reduced pressure by using a rotary evaporator, thereby resulting in Intermediate 3-2 as a solid (amount 2.7 g, yield 85 %).

### (3) Synthesis of Compound 3

Under an argon atmosphere, Intermediate 2-2 (2.7 g, 5. 7 mmol), pyrene-1-boronic acid (3.5 g, 14.2 mmol), tetrakis(triphenylphosphine)palladium (0.4 g, 0.3 mmol), toluene (20 mL), and a 2-M aqueous solution of sodium carbonate (17 mL, 34 mmol) were added to a 200-mL three-necked flask, and the whole was heated at 80°C for 9 hours. Water (100 mL) was added to the reaction liquid in such a manner that a solid would be precipitated. Then, the solid was filtered. The resultant was purified by means of silica gel column chromatography (amount 2.2 g, yield 54 %). The purified product was identified as Compound 3 on the basis of ¹H-NMR and FD-MS. The measured value of the FD-MS was 710.

### Synthesis Example 4 (synthesis of 9,9-dimethyl-2,7-bis(4-(1-pyrenyl)phenyl) (Compound 4))

Compound 4 was synthesized as described below.

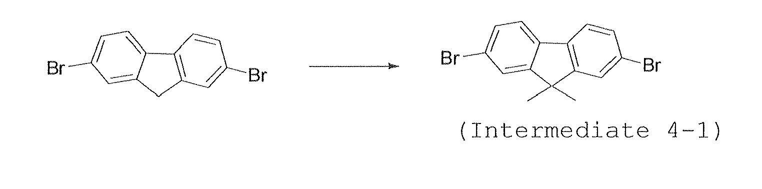

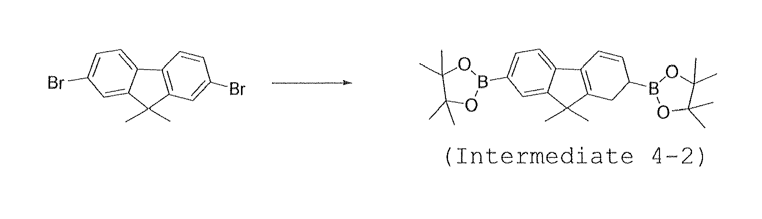

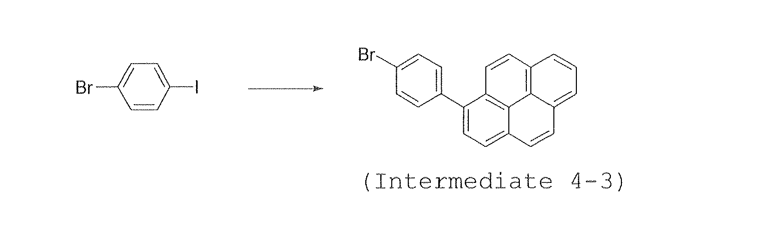

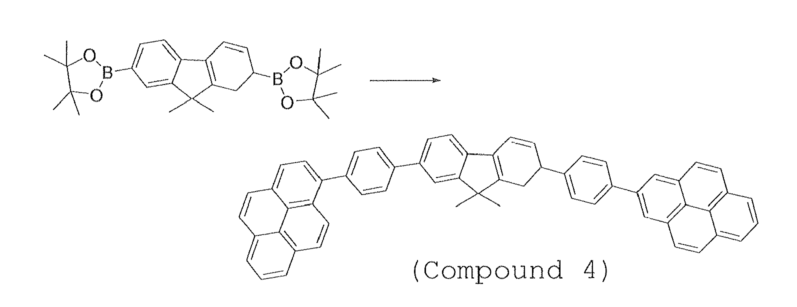

### (1) Synthesis of Intermediate 4-1

2, 7-dibromofluorene (2.0 g, 6.2mmol), dimethyl sulfoxide (0.2 ml), benzyltriethylammonium chloride (0. 07 g, 0.3 mmol), and a 50 wt% aqueous solution of NaOH (2 g) were added to a flask. Next, iodomethane (2.2 g, 15 mmol) was dropped to the mixture, and the whole was stirred for 30 minutes. Water (100 mL) and toluene (100 mL) were added to the reaction liquid in such a manner that an organic layer would be separated. The organic layer was washed with a saturated brine, and was then dried with anhydrous magnesium sulfate. The resultant was concentrated under reduced pressure by using a rotary evaporator, and then the residue was purified by means of silica gel column chromatography, whereby Intermediate 4-1 was obtained (amount 2.0 g, yield 94 %).

### (2) Synthesis of Intermediate 4-2

Under an argon atmosphere, Intermediate 4-1. (2.0 g, 5.8 mmol), bis (pinacolatodiboron) (4.4 g, 17. mmol), a palladium (II) chloride diphenylphosphinoferrocenium·methylene chloride complex (1:1) (0.14 g, 0.2 mmol), potassium acetate (3.4 g, 35 mmol) and dimethyl sulfoxide (40 mL) were added to a 200-mL three-necked flask, and the whole was stirred at 80°C for 9 hours. Water (100 mL) was added to the reaction liquid in such a manner that a solid would be precipitated. Then, the solid was dried under reduced pressure. The solid was purified by means of silica gel column chromatography, whereby Intermediate 4-2 was obtained (amount 1.2 g, yield 47 %).

### (3) Synthesis of Intermediate 4-3

Under an argon atmosphere, 4-bromoiodobenzene (2. 8 g, 10 mmol), pyrene-1-boronic acid (3.0 g, 12 mmol), tetrakis (triphenylphosphine)palladium (0.35 g, 0.3 mmol), toluene (20 mL), and a 2-M aqueous solution of sodium carbonate (15 mL, 30 mmol) were added to a 200-mL three-necked flask, and the whole was heated at 80°C for 8 hours. Water (100 mL) was added to the reaction liquid in such a manner that a solid would be precipitated. Then, the solid was filtered. The resultant was purified by means of silica gel column chromatography, whereby Intermediate 4-3 was obtained (amount 2.8 g, yield 79 %).

### (4) Synthesis of 9,9-dimethyl 2,7-bis(4-(1-pyrenyl)phenyl) (Compound 4)

Under an argon atmosphere, Intermediate 4-2 (1.2 g, 2.7 mmol), Intermediate 4-3 (2.8 g, 7.9 mmol), tetrakis(triphenylphosphine)palladium (0.23 g, 0.2 mmol), toluene (20 mL), and a 2-M aqueous solution of sodium carbonate (8 mL, 16 mmol) were added to a 200-mL three-necked flask, and the whole was heated at 80°C for 8 hours. Water (100 mL) was added to the reaction liquid in such a manner that a solid would be precipitated. Then, the solid was filtered. The resultant was purified by means of silica gel column chromatography (amount 1.36 g, yield 67 %). The purified product was identified as Compound 4 on the basis of ¹H-NMR and FD-MS. The measured value of the FD-MS was 746.

### Synthesis Example 5 (synthesis of 1,4-bis(9,9-di-methyl-'7-(I-pyrenyl)fluoren-2-yl-) (Compound 5))

Compound 5 was synthesized as described below.

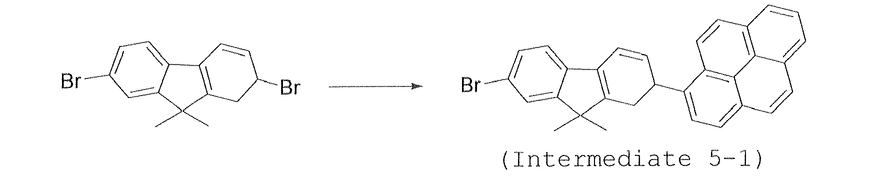

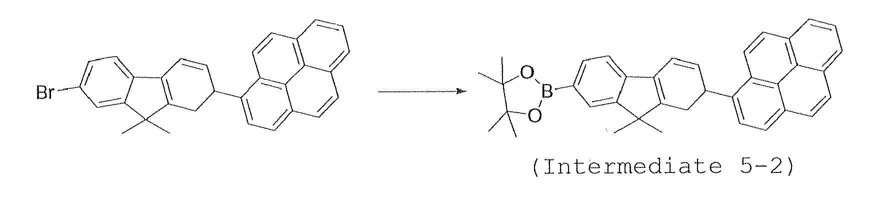

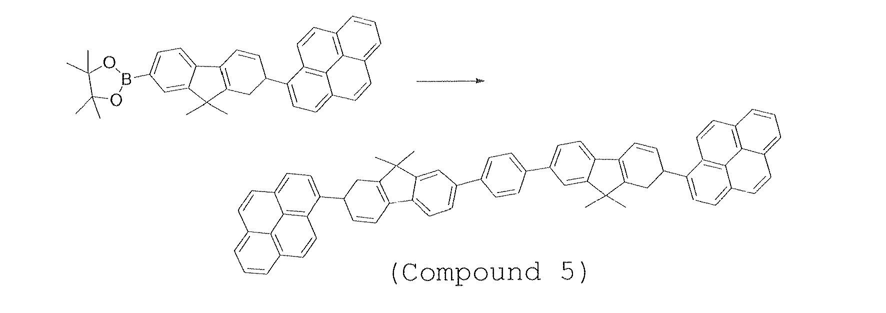

### (1) Synthesis of Intermediate 5-1

Under an argon atmosphere, Intermediate 4-1 (4.0 g, 12. 4 mmol), 1-pyrenylboronic acid (3.0 g, 12 mmol), tetrakis(triphenylphosphine)palladium (0.4 g, 0.4 mmol), toluene (40 mL), and a 2-M aqueous solution of sodium carbonate (18 mL, 36 mmol) were added to a 200-mL three-necked flask, and the whole was heated at 80°C for 8 hours. Water (100 mL) was added to the reaction liquid in such a manner that a solid would be precipitated. Then, the solid was filtered. The resultant was purified by means of silica gel column chromatography, whereby Intermediate 5-1 was obtained (amount 4.2 g, yield 73 %).

### (2) Synthesis of Intermediate 5-2

Under an argon atmosphere, Intermediate 5-1 (4.2g, 9.0 mmol), bis(pinacolatodiboron) (3.44 g, 13.6 mmol), a palladium (II) chloride diphenylphosphinoferrocenium-methylene chloride complex (1:1) (0. 22 g, 0.28 mmol), potassium acetate (2. 6 g, 27 mmol), and dimethyl sulfoxide (40 mL) were added to a 200-mL three-necked flask, and the whole was stirred at 80°C for 9 hours. Water (100 mL) was added to the reaction liquid in such a manner that a solid would be precipitated. Then, the solid was dried under reduced pressure. The solid was purified by means of silica gel column chromatography, whereby Intermediate 5-2 was obtained (amount 2.4 g, yield 50 %).

### (3) Synthesis of 1,4-bis (9,9-dimethyl-7-(1-pyrenyl)fluoren-2-yl) (Compound 5)

Under an argon atmosphere, Intermediate 5-2 (2.4 g, 4.4 mmol), 1,4-diiodobenzene (0.66 g, 2.0 mmol), tetrakis(triphenylphosphine)palladium (0.22 g, 0.2 mmol), toluene (20 mL), and a 2-M aqueous solution of sodium carbonate (6 mL, 12 mmol) were added to a 200-mL three-necked flask, and the whole was heated at 80°C for 8 hours. Water (100 mL) was added to the reaction liquid in such a manner that a solid would be precipitated. Then, the solid was filtered. The resultant was purified by means of silica gel column chromatography (amount 1.2g, yield 69 %) . The purified product was identified as Compound 5 on the basis of ¹H-NMR and FD-MS. The measured value of the FD-MS was 862.

### Synthesis Example 6 (synthesis of 9,9-dimethyl-2,7-bis(4-(N-carbazolyl)phenyl) (Compound 6))

Compound 6 was synthesized as described below.

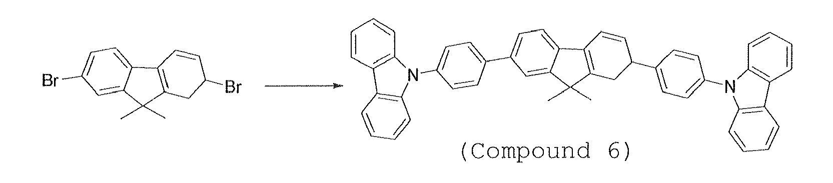

Under an argon atmosphere, Intermediate 4-1 (0.70 g, 2.0 mmol), 4-(N-carbazolyl)phenylboronic acid (1.26 g, 4.4 mmol), tetrakis(triphenylphosphine)palladium (0.14 g, 0.12 mmol), toluene (10 mL), and a 2-M aqueous solution of sodium carbonate (3 mL, 6 mmol) were added to a 200-mL three-necked flask, and the whole was heated at 80°C for 8 hours. Water (100 mL) was added to the reaction liquid in such a manner that a solid would be precipitated. Then, the solidwas filtered. The resultant was purified by means of silica gel column chromatography (amount 1.0 g, yield 74 %) . The purified product was identified as Compound 6 on the basis of ¹H-NMR and FD-MS. The measured value of the FD-MS was 676.

### Synthesis Example 7 (Synthesis of Compound 7)

Compound 7 was synthesized as described below.

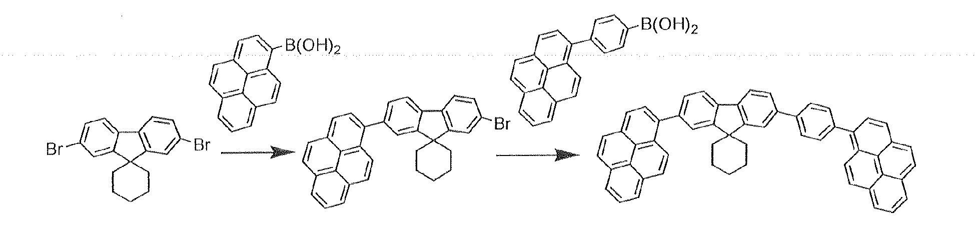

Intermediate (8-1) was synthesized in an amount of 3.54 g and a yield of 69 % by performing the same operation as that of Example 1 (3) except that Intermediate 5-1 described above (3.92 g, 10.0 mmol), 1-pyreneboronic acid (2.46 g, 10.0 mmol), tetrakis (triphenylphosphine) palladium (0.2.3 g, 0.20 mmol), N,N-dimethylformamide (20 mL), and 15 mL of an aqueous solution of potassium carbonate (4.15 g, 30. 0 mmol) were used under an argon atmosphere.
Subsequently, Compound 8 was synthesized in an amount of 3.70 g and a yield of 80 % by performing the same operation as that of Example 1 (3) except that a reaction liquid containing Intermediate (8-1) (3.33 g, 6.5 mmol), 4-pyrenylbenzeneboronic acid (2.30 g, 7.15 mmol), tetrakis(triphenylphosphine)palladium (0.15 g, 0.13 mmol), N,N-dimethylformamide (20mL), and 10 mL of an aqueous solution of potassium carbonate (2.70 g, 19.5 mmol) with one another was used.

### Example 1 (production of organic EL device)

A glass substrate with an ITO transparent electrode measuring 25 mm wide by 75 mm long by 1.1 mm thick (manufactured by GEOMATEC Co. , Ltd.) was subjected to ultrasonic cleaning in isopropyl alcohol for 5 minutes. After that, the substrate was subjected to UV ozone cleaning for 30 minutes. The glass substrate with the transparent electrode line after the washing was mounted on a substrate holder of a vacuum deposition device. First, a 4,4'-bis[N-(1-naphthyl)-N-phenylamino]biphenyl film (α-NPD film) having a thickness of 60 nm was formed on the surface on the side where the transparent electrode line was formed to cover the transparent electrode. The film functions as a hole injecting layer. Further, Compound 1 described above and serving as a host material was deposited from the vapor and formed into a film having a thickness of 40 nm on the hole injecting layer. Simultaneously with the formation, Amine Compound D1 having a styryl group shown below and serving as a light emitting molecule was deposited from the vapor in such a manner that a weight ratio between Amine Compound D1 and Compound 1 would be 3:40. The film functions as a light emitting layer. An Alq film having a thickness of 10 nm was formed on the resultant film. The film functions as an electron injecting layer. After that, Li serving as a reducing dopant (Li source: manufactured by SAES Getters) and Alq shown below were subjected to co-deposition. Thus, an Alq:Li film (having a thickness of 10 nm) was formed as an electron injecting layer (i.e., cathode) . Metal Al was deposited from the vapor onto the Alq:Li film to form a metal cathode. Thus, an organic EL device was produced.
The resultant device was evaluated for current efficiency and for luminance half time upon constant current continuous driving at an initial luminance of 300 cd/m². Table 1 shows the results.

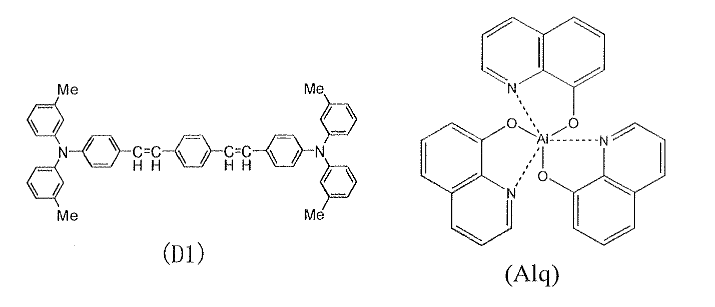

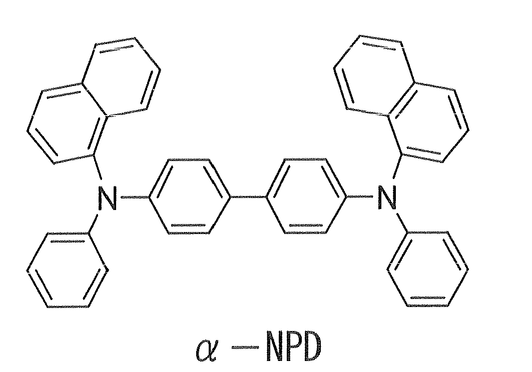

Examples 2 to 6 (production of organic EL devices) Organic EL devices were each produced in the same manner as in Example 1 except that any one of Compounds 2 to 5 and Compound 7 was used instead of Compound 1 as shown in Table 1, and were each evaluated in the same manner as in Example 1. Table 1 shows the results.

### Comparative Examples 1 and 2 (production of organic EL devices)

Organic EL devices were each produced in the same manner as in Example 1 except that Comparative Compound 1 or 2 was used instead of Compound 1 as shown in Table 1, and were each evaluated in the same manner as in Example 1. Table 1 shows the results. Me represents a methyl group.

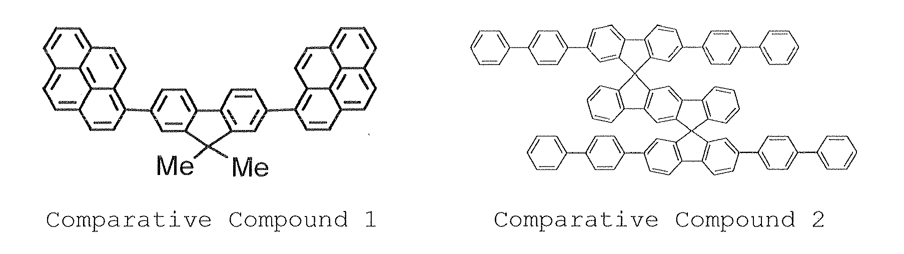

**Table 1**

| | Host material used in light emitting layer | Current efficiency (cd/A) | Luminance half time (hours) | Remark |
|---|---|---|---|---|
| Example 1 | Compound 1 | 10.2 | 11,000 | |
| Example 2 | Compound 2 | 11.3 | 10,000 | |
| Example 3 | Compound 3 | 9.8 | 12,000 | |
| Example 4 | Compound 4 | 9.5 | 13,000 | |
| Example 5 | Compound 5 | 9.4 | 11,500 | |
| Example 6 | Compound 7 | 9.9 | 12,500 | |
| Comparative Example 1 | Comparative Compound 1 | 6.2 | 4,500 | |
| Comparative Example 2 | Comparative Compound 2 | 8.5 | 3,500 | Nonuniform light emission is observed |

As shown in Table 1, Examples 1 to 6 each using a compound of the present invention in a light emitting layer each had a current efficiency higher than those of the compounds used in Comparative Examples 1 and 2, and each showed a luminance half time significantly improved as compared to those of the materials used in the comparative examples. In the case of Comparative Compound 2 used in Comparative Example 2, the organic EL device was observed to emit light nonuniformly. This is probably because Comparative Compound 2 has so high a molecular weight that Comparative Compound 2 is formed into a film while being thermally decomposed upon vapor deposition.

### Example 7 (production of organic EL device)

A glass substrate with an ITO transparent electrode measuring 25 mm wide by 75 mm long by 0.7 mm thick (manufactured by GEOMATEC Co., Ltd.) was subjected to ultrasonic cleaning in isopropyl alcohol for 5 minutes. After that, the substrate was subjected to UV ozone cleaning for 30 minutes. The glass substrate with the transparent electrode after the washing was mounted on a substrate holder of a vacuum deposition device. First, copper phthalocyanine shown below was formed into a film (CuPc film) having a thickness of 10 nm on the surface on the side where the transparent electrode was formed to cover the transparent electrode. The CuPc film functions as a hole injecting layer. An α-NPD film having a thickness of 30 nm was formed on the CuPc film. The α-NPD film functions as a hole transporting layer. Further, Compound 6 described above and serving as a host material was deposited from the vapor and formed into a film having a thickness of 30 nm to serve as a light emitting layer on the α-NPD film. Simultaneously with the formation, bis(2-phenylisoquinolinato)acetylacetonatoiridium (Ir (piq)₂(acac)) shown below was added as a phosphorescent Ir metal complex dopant. The concentration of Ir(piq)₂(acac) in the light emitting layer was set to 15 wt%. The film functions as a light emitting layer. Then, p-biphenylolatobis(2-methyl-8-quinolinolato)aluminum was formed into a film (BAlq film) having a thickness of 10 nm on the resultant film. The BAlq film functions as a hole blocking layer. Further, an Alq film having a thickness of 40 nm was formed on the resultant film. The Alq film functions as an electron injecting layer. After that, LiF as an alkali metal halide was deposited from the vapor to have a thickness of 0.2 nm. Next, aluminum was deposited from the vapor to have a thickness of 150 nm. The resultant Al/LiF film functions as a cathode. Thus, an organic EL device was produced.
The resultant device was subjected to a current test. As a result, the device emitted red light with an emission luminance of 10,000 cd/m² at a voltage of 8 V.

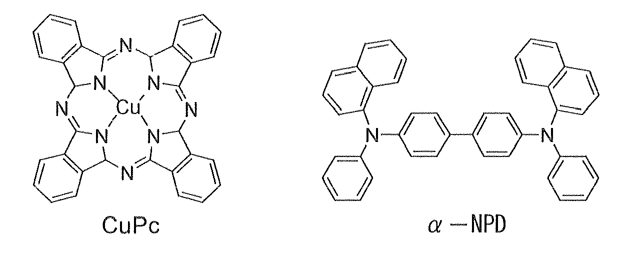

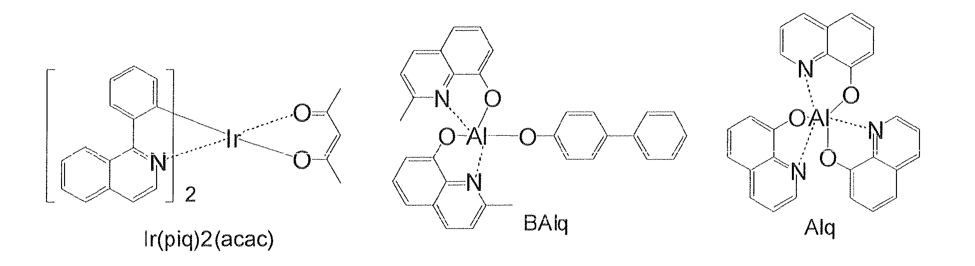

### Synthesis Example 8 (Synthesis of Compound 8)

Compound 8 was synthesized as described below.

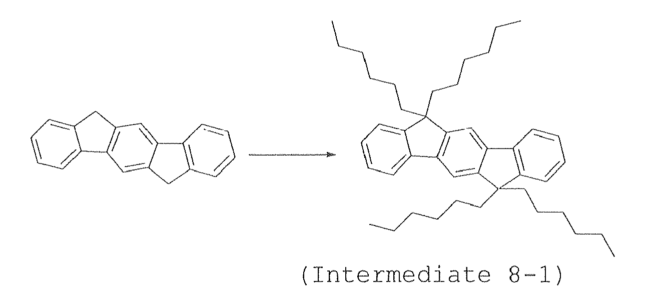

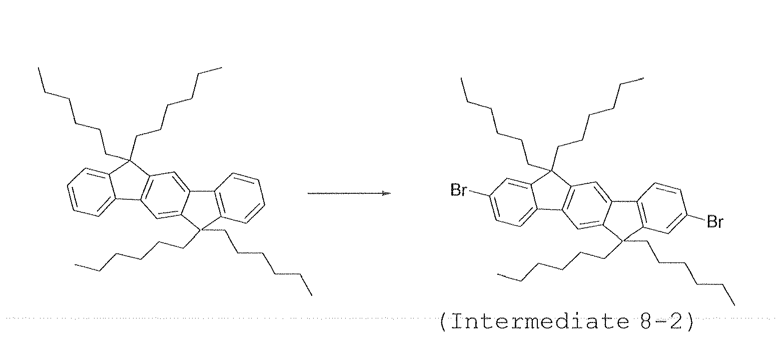

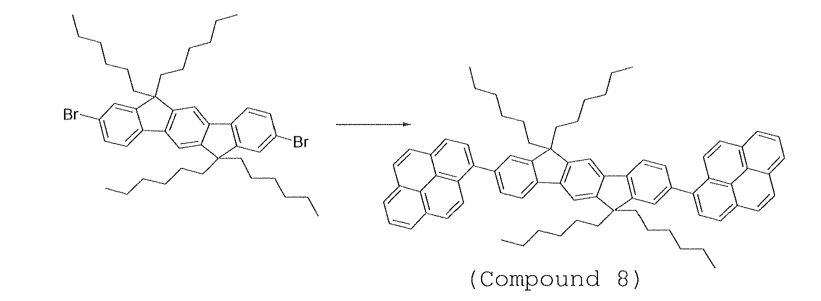

### (1) Synthesis of Intermediate 8-1.

Intermediate 8-1 was synthesized (amount 3.8 g, yield 82 %) in the same manner as in the above item (1) of Synthesis Example 3 except that 1-bromohexane (6.4 g, 39 mmol) was used instead of iodomethane.

### (2) Synthesis of Intermediate 8-2

Intermediate 8-2 was synthesized (amount 3.9 g, yield 80 %) in the same manner as in the above item (2) of Synthesis Example 3 except that Intermediate 8-1 (3.8 g, 6.5 mmol) was used instead of Intermediate 3-1.

### (3) Synthesis of Compound 8

Compound 8 was synthesized (amount 3.3 g, yield 65 %) in the same manner as in the above item (3) of Synthesis Example 3 except that Intermediate 8-2 (3.9 g, 5.2 mmol) was used instead of Intermediate 3-2. The resultant was identified as Compound 8 on the basis of ¹H-NMR and an FD-MS. The measured value of the FD-MS was 990.

### Example 8

A glass substrate with an ITO transparent electrode measuring 25 mm wide by 75 mm long by 1.1 mm thick (manufactured by GEOMATEC Co. , Ltd.) was subjected to ultrasonic cleaning in isopropyl alcohol for 5 minutes. After that, the substrate was subjected to UV ozone cleaning for 30 minutes. Polyethylenedioxy thiophene (PEDOT) was formed into a film having a thickness of 100 nm to serve as a hole injecting layer by a spin coating method on the substrate. Next, a film was formed by using a solution of Compound 8 in dichloroethane on PEDOT by a spin coating method, whereby a light emitting layer was obtained. The light emitting layer had a thickness of 50 nm.
An Alq film having a thickness of 10 nm was formed on the resultant film. The Alq film functions as an electron transporting layer. After that, Li serving as a reducing dopant (Li source: manufactured by SAES Getters) and Alq were subjected to co-deposition. Thus, an Alq: Li film was formed as an electron injecting layer (i.e., cathode). Metal Al was deposited from the vapor onto the Alq:Li film to form a metal cathode. Thus, an organic EL device was formed.
The resultant device was subjected to a current test. As a result, the device emitted blue light, and showed a current efficiency of 4.2 cd/A.

### Example 9

A glass substrate with an ITO transparent electrode measuring 25 mm wide by 75 mm long by 1.1 mm thick (manufactured by GEOMATEC Co. , Ltd.) was subjected to ultrasonic cleaning in isopropyl alcohol for 5 minutes. After that, the substrate was subjected to UV ozone cleaning for 30 minutes. Polyethylenedioxy thiophene (PEDOT) was formed into a film having a thickness of 100 nm to serve as a hole injecting layer by a spin coating method on the substrate. Next, a film was formed by using a solution of Compound 1 produced in Synthesis Example 1 in toluene on PEDOT by a spin coating method, whereby a light emitting layer was obtained. The light emitting layer had a thickness of 50 nm. It should be noted that a dopant represented by the following structural formula was dissolved in the toluene solution in advance in such a manner that the dopant would be added at a ratio of 0.2 to 0.3 mass% with respect to the light emitting layer. 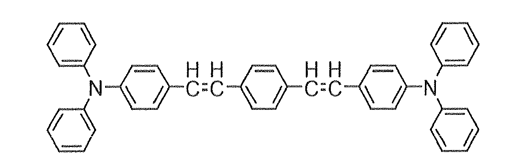 Next, an Alq film having a thickness of 10 nm was formed on the resultant film. The Alq film functions as an electron transporting layer. After that, Li serving as a reducing dopant (Li source: manufactured by SAES Getters) and Alq were subjected to co-deposition. Thus, an Alq:Li film was formed as an electron injecting layer (i.e., cathode). Metal Al was deposited from the vapor onto the Alq:Li film to form a metal cathode. Thus, an organic EL device was formed.
The resultant device was subjected to a current test. As a result, the device emitted blue light, and showed a current efficiency of 9.5 cd/A.

### Comparative Example 3

A device was produced in the same manner as in Example 8 except that Comparative Compound 3 shown below was used instead of Compound 8.
The resultant device was subjected to a current test. As a result, the device emitted blue light with a current efficiency of 2.1 cd/A.

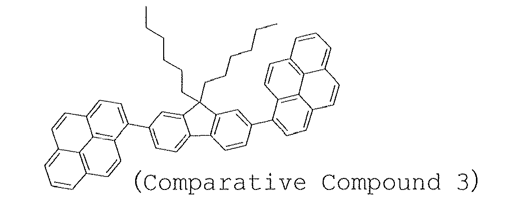

### INDUSTRIAL APPLICABILITY

As described above in detail, the organic EL device using the polycyclic aromatic compound and material for forming a luminescent coating film of the present invention has high luminous efficiency and a long lifetime. Accordingly, the organic EL device is extremely useful as an organic EL device having practical performance.

## Claims

1. A polycyclic aromatic compound represented by the following general formula (1):
X-Y-X' (1)
where:
X and X' each independently represent a substituted or unsubstituted aromatic group having 6 to 50 carbon atoms, or a substituted or unsubstituted aromatic heterocyclic group having 5 to 50 ring atoms, and may be identical to or different from each other; and
Y represents a divalent group containing at least one linking group represented by the following general formula (2) or (3) : 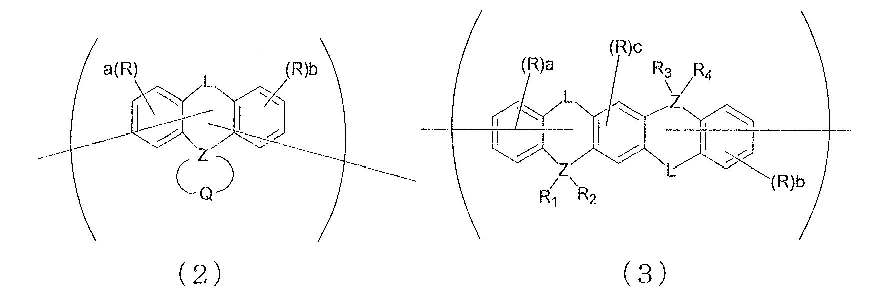
where:
R's and R₁ to R₄ each independently represent a substituted or unsubstituted aromatic group having 6 to 50 ring carbon atoms, a substituted or unsubstituted aromatic heterocyclic group having 5 to 50 ring atoms, a substituted or unsubstituted al kyl group having 1 to 50 carbon atoms, a substituted or unsubstituted alkoxy group having 1 to 50 carbon atoms, a substituted or unsubstituted aralkyl group having 7 to 50 carbon atoms, a substituted or unsubstituted aryloxy group having 5 to 50 ring atoms, a substituted or unsubstituted arylthio group having 5 to 50 ring atoms, a carboxyl group, a halogen atom, a cyano group, a nitro group, or a hydroxyl group, and R₁ and R₂, or R₃ and R₄ may be bonded to each other to form a cyclic structure;
L's each represent a single bond, -(CR'R")_{c}-, -(SiR'R")_{c}-, -O-, -CO-, or -NR'- where R' and R" each independently represent a hydrogen atom, a substituted or unsubstituted aromatic group having 6 to 50 ring carbon atoms, a substituted or unsubstituted aromatic heterocyclic group having 5 to 50 ring atoms, or a substituted or unsubstituted alkyl group having 1 to 50 carbon atoms, c represents an integer of 1 to 10, and R' and R" may be identical to or different from each other;
Z's each represent a carbon atom, a silicon atom, or a germanium atom;
Q represents a cyclic structure forming group; and
a and b each represent an integer of 0 to 4, and c represents an integer of 0 to 2.

2. The polycyclic aromatic compound according to claim 1, wherein, in the general formula (1), at least one of X and X' represents a substituted or unsubstituted aromatic group having 16 to 50 carbon atoms, and Y represents a divalent group containing at least one linking group represented by any one of the following general formulae (4) to (6): 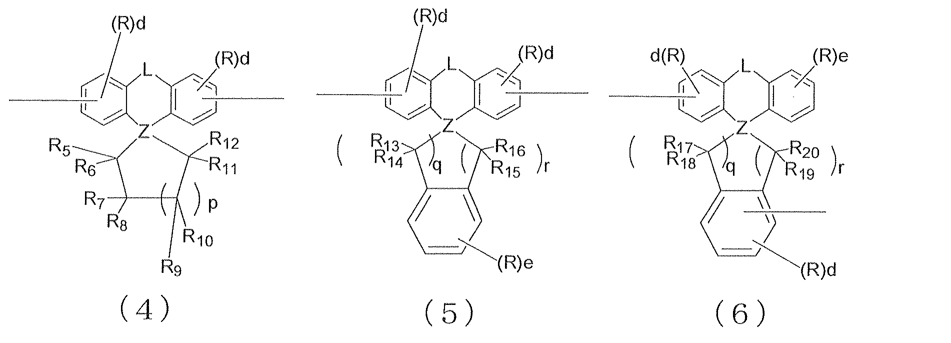 where:
R's and R₅ to R₂₀ each independently have the same meaning as that of each of R's and R₁ to R₄ described above, and two arbitrary adjacent groups among R₅ to R₂₀ may be bonded to each other to form a cyclic structure;
L and Z each independently have the same meaning as that described above;
d's each represent an integer of 0 to 3, and e represents an integer of 0 to 4; and
p, q, and r each represent an integer of 1 to 10.

3. The polycyclic aromatic compound according to claim 1, wherein, in the general formula (1), Y represents a divalent group containing at least one linking group represented by the following general formula (7): 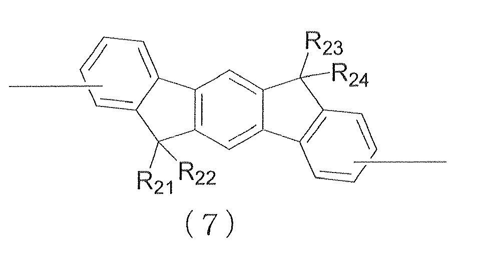 where R₂₁ to R₂₄ each independently have the same meaning as that of each of R's and R₁ to R₄ described above, and R₂₁ and R₂₂, and R₂₃ and R₂₄ may be bonded to each other to form a cyclic structure.

4. The polycyclic aromatic compound according to claim 2, wherein, in the general formula (1), Y represents a linking group represented by the following general formula (8):
- (Ar₁)ₖ-L₁-(Ar₂)ₘ- (L₂)ₙ- (8)
where:
Ar₁ and Ar₂ each independently represent a substituted or unsubstituted, divalent aromatic group having 6 to 12 carbon atoms;
L₁ and L₂ each represent a linking group represented by any one of the general formulae (4) to (6), and L₁ and L₂ may be identical to or different from each other; and
k and m each represent an integer of 0 to 5, and n represents an integer of 0 or 1 provided that k, m, and n never simultaneously represent 0.

5. The polycyclic aromatic compound according to claim 3, wherein, in the general formula (1), Y represents a linking group represented by the following general formula (8'):
-(Ar₁)ₖ-L₁'-(Ar₂)ₘ-(L₂')ₙ- (8')
where:
Ar₁ and Ar₂ each independently represent a substituted or unsubstituted, divalent aromatic group having 6 to 12 carbon atoms;
L₁' and L₂' each represent a linking group represented by the general formulae (7), and L₁' and L₂' may be identical to or different from each other; and
k and m each represent an integer of 0 to 5, and n represents an integer of 0 or 1 provided that k, m, and n never simultaneously represent 0

6. The polycyclic aromatic compound according to any one of claims 1 to 5, wherein, in the general formula (1), at least one of X and X' represents a pyrenyl group or a chrysenyl group.

7. The polycyclic aromatic compound according to any one of claims 1 to 6, wherein the polycyclic aromatic compound comprises a material for an organic electroluminescence device.

8. A material for forming a luminescent coating film, the material comprising an organic solvent solution containing the polycyclic aromatic compound according to any one of claims 1 to 6.

9. An organic electroluminescence device, comprising an organic thin film layer composed of one or multiple layers including at least a light emitting layer, the organic thin film layer being interposed between a cathode and an anode, wherein at least one layer of the organic thin film layer contains the polycyclic aromatic compound represented by the general formula (1) according to claim 1 alone or as a component of a mixture.

10. The organic electroluminescence device according to claim 9, wherein the light emitting layer contains the polycyclic aromatic compound represented by the general formula (1).

11. The organic electroluminescence device according to claim 10, wherein the light emitting layer further contains an arylamine compound and/or a styrylamine compound.

12. The organic electroluminescence device according to claim 10, wherein the light emitting layer further contains at least one kind selected from the group consisting of iridium (Ir), ruthenium (Ru), palladium (Pd), platinum (Pt), osmium (Os), and rhenium (Re).
